(19) Europäisches Patentamt
European Patent Office
Office européen des brevets

(11) **EP 4 438 737 A1**

(12) **EUROPEAN PATENT APPLICATION**

(43) Date of publication:
**02.10.2024 Bulletin 2024/40**

(21) Application number: **23166156.2**

(22) Date of filing: **31.03.2023**

(51) International Patent Classification (IPC):
***C12Q 1/6804*** (2018.01)    ***C12Q 1/6834*** (2018.01)

(52) Cooperative Patent Classification (CPC):
(C-Sets available)
**C12Q 1/6804; C12Q 1/6834**    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Blink AG**
**07747 Jena (DE)**

(72) Inventors:
• **Adelhelm, Karin**
**07749 Jena (DE)**
• **Ellinger, Thomas**
**07745 Jena (DE)**
• **Hubold, Stephan**
**07745 Jena (DE)**
• **Lemuth, Oliver**
**07745 Jena (DE)**
• **Schirrmann, Kerstin**
**07745 Jena (DE)**

• **Schulz, Torsten**
**07745 Jena (DE)**
• **Steinmetzer, Katrin**
**07745 Jena (DE)**
• **Töpfer, Susanne**
**07745 Jena (DE)**
• **Tuchscheerer, Jens**
**07745 Jena (DE)**
• **Ermantraut, Eugen**
**07745 Jena (DE)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **A METHOD OF QUANTIFYING A CONCENTRATION OF AN ANALYTE IN A SAMPLE**

(57)    The present invention relates to a method of quantifying a concentration $c_s$ of an analyte in a sample, and in particular, to a method of quantifying a concentration $c_s$ of a target nucleic acid in a sample based on the principle of random distribution of analytes/targets provided in a sample with a known volume across a defined number of multiple pre-made reaction compartments The present invention also relates to an aqueous suspension of a pre-defined number $N_B$ of hydrogel microspheres for use in a method of quantifying a concentration $c_s$ of an analyte, in particular of a target nucleic acid, in a sample. Moreover, the present invention also relates to a macroparticle for use in such method. Finally, the present invention also relates to a method of generating an aqueous suspension of a pre-defined number $N_B$ of hydrogel microspheres.

**EP 4 438 737 A1**

(52) Cooperative Patent Classification (CPC): (Cont.)

C-Sets
**C12Q 1/6804, C12Q 2527/107, C12Q 2563/143,**
**C12Q 2563/149, C12Q 2563/159;**
**C12Q 1/6834, C12Q 2527/107, C12Q 2563/143,**
**C12Q 2563/149, C12Q 2563/159**

**Description**

FIELD OF THE INVENTION

The present invention relates to a method of quantifying a concentration $c_s$ of an analyte in a sample, and in particular, to a method of quantifying a concentration $c_s$ of a target nucleic acid in a sample. The present invention also relates to the principle of random distribution of analytes/targets in a sample with a known volume across a defined number of multiple pre-made reaction compartments by binding the analytes/targets.

The present invention also relates to an aqueous suspension of a pre-defined number $N_B$ of hydrogel microspheres for use in a method of quantifying a concentration $c_s$ of an analyte, in particular of a target nucleic acid, in a sample. Moreover, the present invention also relates to a macroparticle for use in such method. Finally, the present invention also relates to a method of generating an aqueous suspension of a pre-defined number $N_B$ of hydrogel microspheres.

BACKGROUND OF THE INVENTION

Digital detection assays, for example digital PCR (dPCR) assays, provide for specificity, sensitivity, and precision in target analysis, e.g. nucleic acid analysis. Compared to bulk methods, digital assays, such as dPCR, offer the advantage of absolute quantification without the use of a calibration curve. For this purpose, the assay mix, e.g. amplification mix containing reagents necessary for detecting a target and the analyte/target itself, is divided into thousands of compartments, for example by distributing the liquid across a plurality of wells on a solid substrate. Another approach is the creation of aqueous microdroplets in an immiscible liquid, as for example disclosed in the context of dPCR in Hindson et al., 2011, Anal. Chem., 83, 22, pp. 8604-8610. Quantification of targets in such conventional dPCR is based on the detection of PCR-positive compartments with a known volume.

Hydrogels have been used in the context of dPCR for encapsulating cells (Geng et al., 2014, Anal. Chem. 86, 703-712), and for generating beads with immobilized amplicons for post-PCR analysis (Leng et al., 2010, Lab Chip. 10, 2481-2843). To overcome the inherent need for sophisticated, costly and error-prone microfluidics, hydrogel beads have been also successfully employed for particle templated emulsification (Loncarevic et al., 2021, Plos One, 16, e0242529), serving as mechanical templates for droplet formation in a fluorocarbon oil. There is, however, still a need in the art to facilitate and provide a microfluidic-free digital assay, such as dPCR, that does not rely on compartment volume for quantitation. There is also a need in the art to provide for a digital assay methodology, e.g. dPCR-methodology that is independent of sample volume and provides for target quantification without relying on a precise measure of the volume of the reaction/amplification compartments. There is also a need to provide for a digital assay methodology, e.g. dPCR-methodology, that is easy to perform and that allows to process a wide variety of different sample volumes.

SUMMARY OF THE INVENTION

All these objects are solved by a method of quantifying a concentration $c_s$ of an analyte in a sample, said method comprising the steps:

a)

    a) providing, in any order, an aqueous sample containing, or suspected of containing, an analyte, and a pre-defined number $N_B$ of reaction compartments, said reaction compartments comprising a material capable of binding said analyte;

    b) allowing said reaction compartments to take up aqueous sample and to bind said analyte, preferably all of said analyte, if present in said aqueous sample, by exposing said pre-defined number $N_B$ of reaction compartments to said aqueous sample in a manner which results in a random distribution of analyte across said pre-defined number $N_B$ of reaction compartments, thus resulting in at least some reaction compartments of said pre-defined number $N_B$ of reaction compartments being associated with or comprising said analyte contained by said aqueous sample provided in step a);

    c) optionally, if a flow and/or exchange of aqueous sample between different reaction compartments resulting from step b) is still possible and/or occurs after step b): isolating said reaction compartments resulting from step b) from each other by separating them from each other such that a flow and/or exchange of aqueous sample between different reaction compartments is not possible and does not occur anymore;

d) performing an enzymatic amplification protocol within said reaction compartments, wherein said enzymatic amplification protocol generates a first optically detectable signal in a reaction compartment being associated with or comprising said analyte; and wherein said enzymatic amplification protocol generates a second optically detectable signal in a reaction compartment not being associated with or not comprising said analyte;

e) determining, by detecting said first and second optically detectable signals, respectively, a number $N_{pos}$ of reaction compartments having a first optically detectable signal (= "positive" reaction compartments) and a number $N_{neg}$ of reaction compartments having a second optically detectable signal (= "negative" reaction compartments); wherein the total number of detected reaction compartments $N = N_{pos} + N_{neg}$;

f) calculating a concentration $c_S$ of said analyte in said aqueous sample in accordance with formula

$$c_S = \frac{N_B}{V_S} \cdot -ln\left(\frac{N_{neg}}{N}\right) = \frac{N_B}{V_S} \cdot \lambda;$$

wherein

$c_S$ = concentration of analyte in aqueous sample
$N_B$ = pre-defined number of reaction compartments exposed to aqueous sample
$V_S$ = volume of aqueous sample containing, or suspected of containing, analyte
$N_{neg}$ = number of detected reaction compartments having a second optically detectable signal (= "negative" reaction compartments)
$N$ = number of all detected reaction compartments = $N_{neg} + N_{pos}$.
wherein
$N_{pos}$ = number of detected reaction compartments having a first optically detectable signal (="positive" reaction compartments).

In particular, in one aspect, the present invention relates to a method of quantifying a concentration $c_S$ of an analyte in a sample, said method comprising the steps:

a) providing, in any order, an aqueous sample containing, or suspected of containing, an analyte, and an aqueous suspension of a pre-defined number $N_B$ of hydrogel microspheres, said hydrogel microspheres comprising a material capable of binding said analyte;

b) allowing said hydrogel microspheres to take up aqueous sample and to bind said analyte, preferably all of said analyte, if present in said aqueous sample, by exposing said pre-defined number $N_B$ of hydrogel microspheres to said aqueous sample and mixing said aqueous sample with said suspension of hydrogel microspheres; thus resulting in at least some hydrogel microspheres being associated with or comprising said analyte contained by said aqueous sample provided in step a);

c) transferring said hydrogel microspheres into a liquid phase that is immiscible with water and aqueous solutions, e.g. an oil-phase, thereby generating a suspension of said pre-defined number $N_B$ of hydrogel microspheres in said water-immiscible phase, wherein said hydrogel microspheres are isolated from each other by said water-immiscible liquid phase such that a flow and/or exchange of aqueous sample between different hydrogel microspheres is not possible and does not occur anymore;

d) performing an enzymatic amplification protocol on said hydrogel microspheres, wherein said enzymatic amplification protocol generates a first optically detectable signal in a hydrogel microsphere being associated with or comprising said analyte; and wherein said enzymatic amplification protocol generates a second optically detectable signal in a hydrogel microsphere not being associated with or not comprising said analyte;

e) determining, by detecting said first and second optically detectable signals, respectively, a number $N_{pos}$ of hydrogel microspheres having a first optically detectable signal (= "positive" hydrogel microspheres) and a number $N_{neg}$ of hydrogel microspheres having a second optically detectable signal (= "negative" hydrogel microspheres); wherein the total number of detected hydrogel microspheres $N = N_{pos} + N_{neg}$;

f) calculating a concentration $c_S$ of said analyte in said sample in accordance with formula

$$c_S = \frac{N_B}{V_S} \cdot -ln\left(\frac{N_{neg}}{N}\right) = \frac{N_B}{V_S} \cdot \lambda;$$

wherein

$c_s$ = concentration of analyte in aqueous sample

$N_B$ = pre-defined number of hydrogel microspheres exposed to aqueous sample

$V_S$ = volume of aqueous sample containing, or suspected of containing, analyte

$N_{neg}$ = number of detected hydrogel microspheres having a second optically detectable signal (= "negative" hydrogel microspheres)

$N$ = number of all detected hydrogel microspheres = $N_{neg} + N_{pos}$.

wherein

$N_{pos}$ = number of detected hydrogel microspheres having a first optically detectable signal (="positive" hydrogel microspheres).

In one embodiment, said analyte is:

a) a target nucleic acid; and said enzymatic amplification protocol is a target amplification reaction; or
b) a target protein; and said enzymatic amplification protocol is a signal amplification reaction; or
c) a biological cell or a viral particle or an extracellular vesicle, each comprising various types of nucleic acid within said cell, viral particle or extracellular vesicle; and said enzymatic amplification protocol is a target amplification reaction.

In one embodiment, said analyte is:

a) a target nucleic acid; and said enzymatic amplification protocol is a nucleic acid amplification protocol resulting in specific amplification of said target nucleic acid, if associated with or comprised by any of said hydrogel microspheres, and resulting in a generation of a first optically detectable signal in a hydrogel microsphere, if said hydrogel microsphere comprises or is associated with amplified target nucleic acid; or
b) a target protein; and said enzymatic amplification protocol is a signal amplification reaction involving the formation of an analyte-specific immune-complex on or in a hydrogel microsphere, if said hydrogel microsphere is associated with or comprises said analyte; wherein a chromogenic enzyme is attached to said analyte-specific immune complex, and wherein said enzymatic amplification reaction, through the action of said chromogenic enzyme, furthermore involves an accumulation of an optically detectable product, resulting in a generation of a first optically detectable signal in a hydrogel microsphere, if said hydrogel microsphere comprises or is associated with said analyte; or
c) a target protein; and said enzymatic amplification protocol is a signal amplification reaction involving the formation of an analyte-specific immune-complex on or in a hydrogel microsphere, if said hydrogel microsphere is associated with or comprises said analyte; wherein a nucleic acid label is attached to said analyte-specific immune complex, and wherein said enzymatic amplification reaction furthermore involves a specific nucleic acid amplification of said nucleic acid label, resulting in an accumulation of amplified nucleic acid label and a generation of a first optically detectable signal in a hydrogel microsphere, if said hydrogel microsphere comprises or is associated with said analyte; or
d) a biological cell or a viral particle or an extracellular vesicle, each comprising various types of nucleic acid within said cell, viral particle or extracellular vesicle; and said enzymatic amplification protocol is a nucleic acid amplification protocol resulting in specific amplification of one or several types of nucleic acid comprised within said biological cell, viral particle or extracellular vesicle, if said biological cell, viral particle or extracellular vesicle is associated with or comprised by any of said hydrogel microspheres, and resulting in a generation of a first optically detectable signal in a hydrogel microsphere, if said hydrogel microsphere comprises or is associated with a biological cell, viral particle or extracellular vesicle.

In one embodiment, said material capable of binding said analyte is selected from:

a) cationic polymers, cationic oligomers, cationic monomers, and silica, if the analyte is a nucleic acid, and if said material capable of binding said analyte non-specifically binds nucleic acids;
b) oligonucleotides, if the analyte is a nucleic acid, and if said material capable of binding said analyte specifically binds a particular nucleic acid, e.g. a target nucleic acid; wherein for a given nucleic acid analyte, said oligonucleotide is complementary thereto;
c) antibodies, antibody fragments, and protein receptors, if the analyte is a protein, a biological cell, a viral particle or an extracellular vesicle, and if said material capable of binding said analyte specifically binds a particular protein, e.g. a target protein, or specifically binds a label, tag, prosthetic group or other component associated with said analyte or associated with an antibody, antibody fragment or protein receptor that specifically binds to said analyte.

In one embodiment, said analyte is a nucleic acid, said material capable of binding said analyte binds nucleic acids non-specifically; and wherein said material capable of non-specifically binding nucleic acids is selected from: chitosan and derivatives thereof, gelatin and derivatives thereof, poly(ethyleneimine), poly(2-dimethyl(aminoethyl)methacrylate), poly(lysine), poly(histidine), poly(arginine) and polymer backbones having basic amino acids attached or incorporated as part of such backbone; oligopeptides comprising or consisting of basic amino acid, such as histidine, lysine, and arginine; and monomers selected from basic amino acids, such as histidine, lysine, and arginine.

In one embodiment, said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000.

In one embodiment, said hydrogel microspheres comprise magnetic particles allowing for mechanical handling and transfer of said hydrogel microspheres.

In one embodiment, said method comprises an additional step b*) which is performed after step b) and before step c):

b*) washing said pre-defined number $N_B$ of hydrogel microspheres by exposing them to a wash buffer.

In one embodiment, said method comprises an additional step b**) which is performed

- after step b) and before step c), and,
- if additionally step b*) is performed according to the preceding embodiment (claim 9), then step b**) is performed after step b*) and before step c):

b**) processing said pre-defined number $N_B$ of hydrogel microspheres by exposing them to a solution for performing said enzymatic amplification reaction, said solution comprising

(i) a buffer, mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; or

(ii) a buffer, an analyte-specific antibody or antibody-fragment having a label attached, and a detection reagent for detection of said label; or

(iii) a buffer, an analyte-specific antibody or antibody fragment, a detection antibody for detecting analyte bound to said analyte-specific antibody or antibody fragment, said detection antibody having a label attached, and a detection reagent for detection of said label; wherein in (ii) and (iii), said label is either a nucleic acid tag or a chromogenic enzyme; and,

- if said label is a nucleic acid tag, said detection reagent is a solution comprising mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; and,
- if said label is a chromogenic enzyme, said detection reagent is a solution comprising a substrate for said chromogenic enzyme.

In one embodiment, in step e), said first optically detectable signal generated in said hydrogel microspheres, and optionally also said second optically detectable signal, is detected via a first channel, preferably fluorescence channel, and wherein, further optionally, the total number of detected microspheres $N$ is additionally determined by optical imaging, via a second channel.

In one embodiment, said hydrogel microspheres have an average diameter in a range of from 10 µm to 500 µm, preferably from 20 µm to 500 µm, more preferably from 20 µm to 200 µm, more preferably from 20 µm to 100 µm, and even more preferably from 40 µm to 100 µm .

Ina further aspect, the present invention also relates to a method of quantifying a concentration $c_s$ of a target nucleic acid in a sample, said method comprising the steps:

a) Providing, in any order, an aqueous sample containing, or suspected of containing, a target nucleic acid, and an aqueous suspension of a pre-defined number $N_B$ of hydrogel microspheres, said hydrogel microspheres comprising a material capable of non-specifically binding nucleic acids, including said target nucleic acid, or specifically binding said target nucleic acid;

b) allowing said hydrogel microspheres to take up aqueous sample and to bind said target nucleic acid, preferably all of said target nucleic acid, if present in said aqueous sample, by exposing said pre-defined number $N_B$ of hydrogel microspheres to said aqueous sample and mixing said aqueous sample with said suspension of hydrogel microspheres; thus resulting in at least some hydrogel microspheres of said pre-defined number $N_B$ of hydrogel microspheres being associated with or comprising said target nucleic acid contained by said aqueous sample provided in step a);

c) transferring said hydrogel microspheres into a liquid phase that is immiscible with water and aqueous solutions, e. g. an oil-phase, thereby generating a suspension of said pre-defined number $N_B$ of hydrogel microspheres in said water-immiscible phase, wherein said hydrogel microspheres are isolated from each other by said water-immiscible liquid phase such that a flow and/or exchange of aqueous sample between different hydrogel microspheres is not possible and does not occur anymore;

d) performing a nucleic acid amplification protocol on said hydrogel microspheres that is specific for said target nucleic acid; wherein said nucleic acid amplification protocol results in specific amplification of said target nucleic acid if associated with or comprised by any of said hydrogel microspheres, and resulting in a generation of a first optically detectable signal in a hydrogel microsphere, if said hydrogel microsphere comprises or is associated with amplified target nucleic acid; and wherein said nucleic acid amplification protocol results in no amplification of said target nucleic acid and in a generation of a second optically detectable signal in a hydrogel microsphere, if said hydrogel microsphere is not associated with and does not comprise amplified target nucleic acid;

e) determining, by detecting said first and second optically detectable signals, respectively, a number $N_{pos}$ of hydrogel microspheres having a first optically detectable signal (= "positive" hydrogel microspheres) and a number $N_{neg}$ of hydrogel microspheres having a second optically detectable signal (= "negative" hydrogel microspheres); wherein the total number of detected microspheres $N = N_{pos} + N_{neg}$;

f) calculating a concentration $c_s$ of a target nucleic acid in said aqueous sample in accordance with formula

$$c_S = \frac{N_B}{V_S} \cdot -ln\left(\frac{N_{neg}}{N}\right) = \frac{N_B}{V_S} \cdot \lambda;$$

wherein

$c_s$ = concentration of target nucleic acid in aqueous sample
$N_B$ = pre-defined number of hydrogel microspheres exposed to aqueous sample
$V_S$ = volume of aqueous sample containing, or suspected of containing, target nucleic acid
$N_{neg}$ = number of detected hydrogel microspheres having a second optically detectable signal (= "negative" hydrogel microspheres)
$N$ = number of all detected hydrogel microspheres = $N_{neg} + N_{pos}$;
wherein
$N_{pos}$ = number of detected hydrogel microspheres having a first optically detectable signal (="positive" hydrogel microspheres).

This particular method of quantifying a concentration $c_s$ of a target nucleic acid in a sample as outlined in the preceeding paragraph may also be an embodiment of the (general) method of quantifying a concentration $c_s$ of an analyte in a sample, as described herein.

In yet a further aspect, the present invention also relates to an aqueous suspension of a pre-defined number $N_B$ of hydrogel microspheres for use in a method of quantifying a concentration $c_s$ of an analyte, preferably of a target nucleic acid, in a sample according to the present invention, said aqueous suspension comprising an aqueous solvent and a pre-defined number $N_B$ hydrogel microspheres, wherein said hydrogel microspheres comprise a material capable of binding an analyte, preferably capable of binding a nucleic acid.

In one embodiment of said suspension, said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000.

In yet a further aspect, the present invention also relates to a macroparticle for use in a method of quantifying a concentration $c_s$ of an analyte, preferably of a target nucleic acid, in a sample according to the present invention, such method being as defined herein, said macroparticle comprising a matrix and a pre-defined number $N_B$ of hydrogel microspheres embedded in said matrix of said macroparticle, wherein said macroparticle is dried, preferably freeze-dried.

In one embodiment of said macroparticle, said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000.

In one embodiment of said macroparticle, said matrix comprises or is made of a material that is an excipient for drying processes, in particular for freeze drying.

In one embodiment of said macroparticle, said excipient for drying, in particular for freeze drying, is selected from saccharides, such as trehalose, sucrose, mannitol, glucose, fructose, lactose, mannitol, inositol, hydroxypropyl-β-cyclodextrin and combinations thereof; polymers such as polyethyleneglycol (PEG), polyvinylpyrrolidone (PVP), dextran, gelatin; amino acids, such as arginine, histidine, and glycine; and combinations of any of the foregoing

In one embodiment of said macroparticle, said macroparticle is substantially spherical, globular, droplet-shaped, ellipsoidal or otherwise round-shaped and has an average diameter in a range of from 1 mm to 50 mm, preferably from 2 mm to 20 mm, more preferably from 2 mm to 15 mm, and even more preferably from 2 mm to 10 mm, and even more preferably from 2 mm to 5 mm.

The objects of the present invention are also solved by a method of generating a macroparticle according to the present invention, said method comprising the steps:

a) providing, in any order, a suspension of a predefined number $N_B$ of hydrogel microspheres, preferably an aqueous suspension of a predefined number $N_B$ of hydrogel microspheres, as defined herein, an aqueous solvent and at least one matrix material as defined herein;

b) adding said at least one matrix material to said aqueous solvent, thereby dissolving said matrix material in said aqueous solvent, and mixing said solution of matrix material with said suspension, preferably aqueous suspension, of a predefined number $N_B$ of hydrogel microspheres, thereby forming a suspension of said hydrogel microspheres in said aqueous solvent;

c) generating droplets of said suspension formed in step b), such droplets having a defined size and volume, and dispensing such droplets into a water-immiscible liquid phase, e.g. an oil phase that is at a temperature in a range of from -100°C to -10°C, preferably from - 90°C to -20°C, more preferably from -80°C to -30°C; thereby allowing said droplets to freeze;

d) separating said frozen droplets from said water-immiscible liquid phase and drying, preferably freeze-drying, them, thus resulting in substantially spherical, globular, droplet-shaped, ellipsoidal or otherwise round-shaped macroparticles according to the present invention.

In one embodiment of the method of generating a macroparticle, the aqueous solvent that is or may be part of the suspension of a predefined number $N_B$ of hydrogel microspheres provided in step a), is the same as the aqueous solvent also, but separately, provided in step a) which is subsequently used in step b) to dissolve said at least one matrix material.

In an embodiment of said method of generating a macroparticle according to the present invention, the matrix material may also or alternatively be provided in step a) together with said aqueous solvent, i.e. as an aqueous solution of matrix material dissolved within said aqueous solvent (instead of being provided (e.g. as a solid) separately from said aqueous solvent in step a)); and then step b) does not require dissolving matrix material in said aqueous solvent anymore, but merely comprises mixing the aqueous solution of matrix material already provided in step a), with said suspension, preferably with said aqueous suspension, of a predefined number $N_B$ of hydrogel microspheres, thereby forming a suspension of said hydrogel microspheres in said aqueous solvent.

According to this embodiment, the method of generating a macroparticle according to the present invention comprises the steps:

a) providing, in any order, a suspension of predefined number $N_B$ of hydrogel microspheres, preferably an aqueous suspension of a predefined number $N_B$ of hydrogel microspheres, as defined herein, and an aqueous solution of at least one matrix material, as defined herein, dissolved within an aqueous solvent;

b) mixing said aqueous solution of said at least one matrix material with said suspension, preferably aqueous suspension, of a predefined number $N_B$ of hydrogel microspheres, thereby forming a suspension of said hydrogel microspheres in said aqueous solvent;

c) generating droplets of said suspension formed in step b), such droplets having a defined size and volume, and dispensing such droplets into a water-immiscible liquid phase, e.g. an oil phase that is at a temperature in a range of from -100°C to -10°C, preferably from - 90°C to -20°C, more preferably from -80°C to -30°C; thereby allowing said droplets to freeze;

d) separating said frozen droplets from said water-immiscible liquid phase and drying, preferably freeze-drying, them, thus resulting in substantially spherical, globular, droplet-shaped, ellipsoidal or otherwise round-shaped macroparticles according to the present invention.

In one specific embodiment of the foregoing embodiment of the method of generating a macroparticle, the aqueous solvent that is or may be part of the suspension of a predefined number $N_B$ of hydrogel microspheres provided in step a), is the same as the aqueous solvent which is or forms part of the aqueous solution of said at least one matrix material dissolved therein.

In a further aspect, the present invention relates to a method of generating an aqueous suspension of a pre-defined number $N_B$ of hydrogel microspheres as defined herein, according to the present invention, said method comprising the step:

a) Providing a macroparticle as defined herein according to the present invention and dissolving such macroparticle in an aqueous solvent; or
a*) providing a predefined number $N_B$ of hydrogel microspheres, as defined herein, in dried form, and suspending them in an aqueous solvent.

DETAILED DESCRIPTION OF THE INVENTION

The present inventors have surprisingly found that it is possible to perform digital amplification reactions, such as digital PCR, without requiring a precise determination of the volume of the compartments used during the performance of such digital amplification. Instead, only the respective number of compartments used for amplification must be known. Likewise, in accordance with the present invention it is not necessary to adjust sample volumes or sample concentrations before performing quantitative determinations. The present invention provides for an easy methodology to perform digital amplification reactions and enrichment of target analyte(s) from different sample(s) or sample volume(s) without relying on a precise measure of volumes of amplification compartments. Moreover, unlike classical digital amplification methods, the present invention allows for usage of an entire sample volume (for example resulting from a nucleic acid elution protocol) without having to adjust such sample volume or analyte concentration first. The methodology according to the present invention can be rapidly performed, does not require sophisticated microfluidic set-up or instrumentation and can also be easily integrated into existing laboratory hardware or automation processes.

Whilst herein, sometimes reference is made to digital PCR as an example of a target amplification reaction, it is clear that the methodology according to the present invention also includes and refers to signal amplification reactions, for instance as frequently used in enzymatic immunoassays.

It should also be noted that aspects of the present invention can be performed with any kind of reaction compartment that is capable of taking up a liquid sample and that is equipped with and comprises a material capable of binding an analyte from a sample, such material being for example in the form of a binding member. Such reaction compartment suitable for the present invention is also equipped with and provides for a volume that is suitable for performing an enzymatic or chemical reaction, e.g. an enzymatic amplification reaction, for detecting an analyte previously bound by said material, e.g. by said binding member.

Hence, the term "reaction compartment", as used herein preferably refers to any entity that comprises a material capable of binding an analyte and that comprises and provides for a volume that is suitable for taking up, and preferably encompassing, an aqueous sample (or a fraction thereof), such aqueous sample containing, or suspected of containing, an analyte; wherein in such volume of said reaction compartment also an enzymatic or chemical detection reaction, e.g. an enzymatic amplification reaction, for detecting said analyte, can be (subsequently) performed. Although, the volume and dimensions of such a suitable reaction compartment are not relevant for determining the concentration of an analyte in accordance with the present invention (but only their pre-defined number $N_B$, which is exposed to an aqueous sample, as becomes clear from the calculation formula used according to the present invention), typically, the preferred dimensions of a "reaction compartment" in accordance with embodiments of the present invention are chosen such that they are in the micrometer range, e.g. that their longest dimension is < 1mm, preferably $\leq 500\mu m$, more preferably $\leq 300\mu m$, more preferably $\leq 200\mu m$, even more preferably $\leq 150\mu m$, and even more preferably $\leq 100\mu m$. Because such reaction volume of the reaction compartments, e.g. of the hydrogel microspheres, e.g. nanoreactor beads or nanoreactor microspheres, is of a magnitude in the nanoliter range, they are sometimes also simply referred to as "nanoreactors". For example hydrogel microspheres with a mean diameter of 500 micrometers, 100 micrometers and 20 micrometers respectively have a volume of 65 nanoliters, 0.5 nanoliters and 0.004 nanoliters, respectively.

Examples of a "reaction compartment", in accordance with embodiments of the invention are:

- a well, chamber, channel, recess, groove or trench on a solid substrate;
- a vessel or container or pot, preferably having dimensions in the micrometer range;
- a solid particle with an inner volume that is accessible from the outside; for example having an interstitial pore space or a single interior volume accessible from the outside;
- a protrusion or projection affixed on a solid substrate (e.g. a "spot" on a substrate), such protrusion or projection having an interstitial pore space allowing for the take up of an aqueous sample;
- a porous particle, e.g. a hydrogel particle having pores allowing for the take up of an aqueous sample.

In performing embodiments of the method of quantifying in accordance with the present invention, it is to be observed that a pre-defined number $N_B$ of reaction compartments are allowed to take up aqueous sample and to bind said analyte, preferably all of said analyte, if present in said aqueous sample, by exposing said pre-defined number $N_B$ of reaction compartments to said aqueous sample in a manner which results in a random distribution of analyte across said pre-defined number $N_B$ of reaction compartments, thus resulting in at least some reaction compartments of said pre-defined number $N_B$ of reaction compartments being associated with or comprising said analyte contained by said aqueous sample.

It should be noted that a random distribution of analyte across said pre-defined number $N_B$ of reaction compartments can be achieved in different ways, depending on the type of reaction compartment used. For example if said reaction compartments are wells or recesses on a solid substrate, a random distribution of analyte (and of aqueous sample) can be achieved by fully immersing said substrate in said aqueous sample and ensuring a random flow and distribution of aqueous sample over said substrate, thus allowing for an equally probable exposure of each reaction compartment to said aqueous sample.

If said reaction compartments are, however, particles which are not affixed to a substrate, i.e. they can be distributed freely in a volume of liquid, e.g. small particles, vessels, in particular porous particles, e.g. hydrogel particles or hydrogel microspheres having pores allowing for the take up of an aqueous sample, or solid particles with an inner volume that is accessible from the outside; for example having an interstitial pore space or a single interior volume accessible from the outside, then a random distribution of analyte (and of aqueous sample) can be achieved by mixing an aqueous suspension of said (porous) particles and said aqueous sample thereby exposing all of said (porous) particles to said aqueous sample (and analyte if present therein).

In embodiments where the reaction compartments are porous particles, it should be noted that such particles typically have a pore space, sometimes also referred to as an "interstitial pore space" that is accessible from the outside of the particle and that allows for the take up of liquid, e.g. of an aqueous sample, from the surroundings of said particle through capillary action. When these particles are in a dry state and become exposed to and are brought in contact with water, an aqueous solution or an aqueous sample, they imbibe or resorb such liquid, similar to a sponge. If they have already been wetted before and therefore already contain some liquid, for example if they are provided in an aqueous suspension, they can nevertheless still be subsequently exposed to an aqueous sample containing, or suspected of containing, analyte, by mixing said particles (as part of a suspension) with said aqueous sample. Because of accessibility of the pore space from the outside of the particles, they will take up aqueous sample and therefore also analyte, if present in said sample, because of such exposure. Taking hydrogel microspheres as an example, the hydrogel that forms part of these particles, typically and preferably is porous, and if such hydrogel microsphere is exposed to an aqueous sample containing, or suspected of containing, analyte, there will be an exchange of liquid from and to the pore space of the hydrogel microsphere, as a result of which aqueous sample and analyte, if present therein, will be taken up by the porous hydrogel microsphere.

It should be noted that in accordance with one important aspect of the present invention, the use of a pre-defined number $N_B$ of reaction compartments, as defined herein, and the use of information about the sample volume, about the number of reaction compartments being associated with or comprising said analyte (after the pre-defined number $N_B$ of reaction compartments has been exposed to said aqueous sample), and about the number of reaction compartments not being associated with or comprising said analyte (again, after the pre-defined number $N_B$ of reaction compartments has been exposed to said aqueous sample), fully suffices to determine and quantify the concentration of analyte within the sample, in accordance with the formula (herein also sometimes referred to as the "calculation formula"):

$$c_S = \frac{N_B}{V_S} \cdot -ln\left(\frac{N_{neg}}{N}\right) = \frac{N_B}{V_S} \cdot \lambda;$$

wherein

$c_s$ = concentration of analyte in aqueous sample
$N_B$ = pre-defined number of reaction compartments exposed to aqueous sample
$V_S$ = volume of aqueous sample containing, or suspected of containing, analyte
$N_{neg}$ = number of detected reaction compartments having a second optically detectable signal (= "negative" reaction compartments)
$N$ = number of all detected reaction compartments = $N_{neg}$ + $N_{pos}$.
wherein
$N_{pos}$ = number of detected reaction compartments having a first optically detectable signal (="positive" reaction compartments).

A knowledge of other factors such as volume of reaction compartments is not necessary. Moreover, the present invention works with a wide range of sample volumes, and it should only be ensured that the volume of the sample Vs containing, or suspected of containing, the analyte, should be known. Hence the present invention is extremely versatile and can be applied to almost any sample and sample volume by choosing an appropriate pre-defined number $N_B$ of reaction compartments which become exposed to the sample under investigation. Hence, embodiments of the present invention work with any reaction compartment that is capable and suitable for

- binding an analyte from an aqueous sample;
- providing a volume and hence a reaction chamber for performing a chemical/enzymatic detection of bound analyte, by means of an enzymatic amplification reaction;
- providing and holding reagents in such reaction chamber which reagents allow to perform an enzymatic amplification reaction that is analyte-specific;
- releasing such reagents and allowing them to form a solution for performing said enzymatic amplification reaction (if they are not already in such solution form), after reaction compartments have become isolated from one another,
- carrying out the enzymatic amplification reaction in the reaction compartment.

As used herein, the term "microsphere", refers to spherical bodies, or substantially spherical, globular, droplet-shaped, ellipsoidal or otherwise round-shaped, e. g. egg-shaped, bodies, having an average diameter in a range of from 10 $\mu$m to 500 $\mu$m, preferably from 20 $\mu$m to 500 $\mu$m, more preferably from 20 $\mu$m to 200 $\mu$m. more preferably from 20 $\mu$m to 100 $\mu$m and even more preferably from 40 $\mu$m to 100 $\mu$m. The term "hydrogel microsphere", as used herein, refers to microspheres that form a hydrogel, in particular a porous hydrogel upon contact with, or when being exposed to, or when comprising, an aqueous solution. Such hydrogel microsphere is preferably "porous" in the sense that it has pores that allow for the take up of an aqueous sample, preferably through capillary forces. In one embodiment, the average pore size of such hydrogel microsphere is < 100 nm, preferably < 50 nm, more preferably <25 nm, more preferably < 15 nm, and even more preferably < 10 nm

An example of suitable hydrogel microspheres that may be used in the context of the present invention are hydrogel microspheres as disclosed in International Patent Application No. PCT/EP2022/080978. An example of these particular hydrogel microspheres are microspheres comprising agarose as a first material which is capable of forming a porous hydrogel, and cross-linked chitosan as a second material which forms a network within said porous hydrogel formed by said first material. However, as outlined herein, hydrogel microspheres in accordance with the present invention may also be made of different materials, as long as they are capable of forming a hydrogel and as long as they comprise a material that is capable of binding an analyte.

In one embodiment, said material capable of binding said analyte is selected from:

a) cationic polymers, cationic oligomers, cationic monomers, and silica, if the analyte is a nucleic acid, and if said material capable of binding said analyte binds nucleic acids non-specifically;
b) oligonucleotides, if the analyte is a nucleic acid, and if said material capable of binding said analyte specifically binds a particular nucleic acid, e.g. a target nucleic acid; wherein for a given nucleic acid analyte, said oligonucleotide is complementary thereto;
c) antibodies, antibody fragments, and protein receptors, if the analyte is a protein, a biological cell, a viral particle or an extracellular vesicle, and if said material capable of binding said analyte specifically binds a particular protein, e.g. a target protein, or specifically binds a label, tag, prosthetic group or other component associated with said analyte or associated with an antibody, antibody fragment or protein receptor that specifically binds to said analyte.

In a preferred embodiment, wherein said analyte is a nucleic acid, said material capable of binding said analyte binds nucleic acids non-specifically; and said material capable of non-specifically binding nucleic acids is selected from: chitosan and derivatives thereof, gelatin and derivatives thereof, poly(ethyleneimine), poly(2-dimethyl(aminoethyl)methacrylate), poly(lysine), poly(histidine), poly(arginine) and polymer backbones having basic amino acids attached or incorporated as part of such backbone; oligopeptides comprising or consisting of basic amino acid, such as histidine, lysine, and arginine; and monomers selected from basic amino acids, such as histidine, lysine, and arginine.

It should be noted in this context that the term "peptide" or "oligopeptide", as used herein, is meant to refer to a polymer/oligomer of amino acids linked to each other via peptide bonds. In one embodiments, such peptide bonds are exclusively peptide bonds formed between the carboxyl group on the $\alpha$-carbon atom of one amino acid residue to the amine-nitrogen on the $\alpha$-carbon atom of a next amino acid residue, i.e. through a condensation reaction involving the respective functional groups (amino- and carboxyl-groups) attached to the $\alpha$-carbon atoms of their respective amino acid residues, forming a classical peptide/protein backbone. Such type of peptide bond is the classical peptide bond also sometimes referred to as "eupeptide bond". In another embodiment, the term "peptide" or "oligopeptide", as used herein, may, however, also refer to a polymer/oligomer of amino acids wherein some or all of the amino acids are linked to each other via peptide bonds formed through condensation reactions involving respective functional groups that are part of the sidechains of amino acids (rather than being attached to the respective $\alpha$-carbon atoms of their respective amino acid residues). Such type of peptide bond is also sometimes referred to as "isopeptide bond" and means that at least one of the functional group partners involved therein are part of a sidechain of an amino acid residue. As an example, the ε-amino-group of lysine may form a peptide bond with the α-carboxy-group of a glutamic acid or with the γ-carboxy-group thereof. Both of these peptide bonds would qualify as an "isopeptide bond".

The terms "reaction compartment/hydrogel microsphere" and "reaction compartments/hydrogel microspheres", as used herein is meant to refer to a "reaction compartment" or "reaction compartments", as defined herein, in general, and in particular to a "hydrogel microsphere" or "hydrogel microspheres", as defined herein, as a preferred embodiment.

The term "analyte" as used herein, refers to a nucleic acid of interest, a protein of interest, a biological cell of interest or a virus of interest that is to be detected and quantified in a sample. However, in the case of the analyte being a biological cell of interest, it should be noted that such biological cell comprises a particular protein or particular nucleic acid that can be used for detection purposes of the biological cell and that may act as representative analyte for such biological cell. Similarly, also a virus of interest may comprise a protein or a nucleic acid that can be used for detection purposes and that may act as a representative analyte for such virus.

The term "target nucleic acid" or "target protein", as used herein, refers to a nucleic acid or protein, respectively that is the analyte of interest, the concentration of which is to be quantified in a sample.

The term "pre-defined number $N_B$ of reaction compartments/hydrogel microspheres" refers to an absolute numerical value which denotes the total number of hydrogel microspheres that are provided in step a) of the method of quantifying according to the present invention. It is important to note that such number is "pre-defined" in the sense that the exact number of reaction compartments/hydrogel microspheres which are provided in step a) of a method of quantifying a concentration $c_s$ of an analyte in a sample in accordance with the present invention, is known and/or has been adjusted to a known value.

Because reaction compartments/hydrogel microspheres in accordance with the present invention comprise a material that is capable of binding the analyte, it is possible, in step b) of the method of quantifying a concentration, that the reaction compartments/hydrogel microspheres bind the analyte when being mixed with an aqueous sample containing, or suspected of containing, an analyte. As a result of such mixing step, the reaction compartments/hydrogel microspheres in accordance with the present invention are exposed to said sample and, if present, to said analyte, and as a result thereof become associated with or comprise said analyte.

The phrase "reaction compartments/microsphere is associated with or comprises said analyte" as used herein, refers to a scenario wherein a reaction compartment/microsphere has an analyte in its spatial proximity and/or has such analyte incorporated and/or has such analyte bound. If subsequently such reaction compartment/hydrogel microsphere that is associated with or comprises said analyte, is transferred into a water-immiscible phase such analyte cannot dissociate from said reaction compartment/hydrogel microsphere. In a preferred embodiment, the term "is associated with or comprises said analyte" is used synonymously with the term "is bound with said analyte" or "is bound to said analyte". In one embodiment, the term "is associated with or comprises said analyte" is used synonymously with the term "has analyte enriched in its spatial proximity and/or has such analyte incorporated and/or has such analyte bound".

The term "signal or target amplification reaction", as used herein, refers to a chemical or biochemical detection reaction, in which either the signal used for detection of the analyte is amplified, or the target (i. e. analyte) itself to be detected and/or quantitated, is first amplified and subsequently detected. Typical examples of a target amplification reaction are nucleic acid amplification reactions, such as polymerase chain reaction (PCR). Typical examples of signal amplification reactions are immunochemistry reactions, such as immunoassays involving the formation of an analyte-specific immune-complex that is subsequently detected.

In accordance with embodiments of the present invention, an enzymatic amplification protocol is performed within reaction compartments which generates a first optically detectable signal in a reaction compartment that is associated with or comprises the analyte, whereas a second optically detectable signal is generated in a reaction compartment that is not associated with or does not comprise the analyte. By means of such first and second optically detectable signals, respectively, different sets of reaction compartments can be determined and distinguished from each other that have or show or display such first or second optically detectable signals, respectively. These two sets of reaction compartments with a first or second optically detectable signal respectively are herein also sometimes referred to as "positive reaction compartments" and "negative reaction compartments", respectively, and they differ from each other in that the "positive reaction compartments" are reaction compartments that are associated with or comprise analyte, whereas "negative reaction compartments" are reaction compartments that are not associated with and do not comprise analyte. The total number of detected reaction compartments is the sum of the positive and negative reaction compartments, i. e. $N = N_{pos} + N_{neg}$.

The term "first optically detectable signal", as used herein, refers to a signal that can be observed by suitable detection means, e.g. an optical detector or optical channel, e.g. a fluorescent channel, and that is specific and indicative of a "positive reaction compartment", i.e. a reaction compartment that is associated with or comprises an analyte. Such "first optically detectable signal" is characterized and defined in terms of its intensity and wavelength which may be a specific intensity and/or wavelength value, respectively, or a defined intensity and wavelength range, respectively. If such "first optically detectable signal is characterized and defined in terms of a defined intensity and wavelength range, respectively, it is preferred that such range(s) is(are) narrow.

The term "second optically detectable signal", as used herein, refers to a signal that can also be observed by suitable detection means, e.g. an optical detector or optical channel, e.g. a fluorescent channel, for example the same detector that is also used for detection of the first optically detectable signal. Such "second optically detectable signal" is specific and indicative of a "negative reaction compartment", i.e. a reaction compartment that is not associated with and does not comprises an analyte.

Such "second optically detectable signal" is firstly and foremostly characterized and defined by the fact that such second optically detectable signal is different from the first optically detectable signal in terms of intensity and/or wavelength (and therefore allows a distinction between "positive" and "negative" reaction compartments, characterized by such "first" and "second" optically detectable signals, respectively). Hence, such "second optically detectable signal" has a different intensity value or range, or a different wavelength value or range, or both, in comparison to the first optically detectable signal. The underlying idea is that a classification and distinction of reaction compartments can be made allowing to determine whether a respective reaction compartment is associated with or comprises an analyte, in which case such reaction compartment is a "positive reaction compartment"; or whether a respective reaction compartment is not associated with, and does not comprise, an analyte, in which case such reaction compartment is a "negative reaction compartment".

In a preferred embodiment, the "second optically detectable signal", as used herein, may be equated and used synonymously with "an optically detectable signal that is different from the first optically detectable signal". In a particularly preferred embodiment, the "second optically detectable signal", as used herein, may be equated and used synonymously with "no first optically detectable signal", i.e. such "second optically detectable signal" is characterized by the absence of a "first optically detectable signal". In such embodiments, then the "first optically detectable signal" would be the only proper optical signal to be detected, i.e. it would be "an optically detectable signal", and the "second optically detectable signal" would simply be "no optically detectable signal".

Therefore, in accordance with a preferred embodiment of the present invention, step d) of the method of quantifying a concentration $c_s$ of an analyte in a sample would be:

d) performing an enzymatic amplification protocol within said reaction compartments, wherein said enzymatic amplification protocol generates a first optically detectable signal in a reaction compartment being associated with or comprising said analyte; and wherein said enzymatic amplification protocol generates no first optically detectable signal in a reaction compartment not being associated with or not comprising said analyte.

In a particularly preferred embodiment of the method of quantifying a concentration $c_s$ of an analyte in a sample, step d) would be:

d) performing an enzymatic amplification protocol within said reaction compartments, wherein said enzymatic amplification protocol generates an optically detectable signal in a reaction compartment being associated with or comprising said analyte; and wherein said enzymatic amplification protocol generates no optically detectable signal in a reaction compartment not being associated with or not comprising said analyte.

In any of the above embodiments, a preferred reaction compartment is hydrogel microsphere.

In accordance with preferred embodiments of the present invention, an enzymatic amplification protocol is performed which generates a first optically detectable signal in a hydrogel microsphere that is associated with or comprises the analyte, whereas a second optically detectable signal, which may be no optically detectable signal or not a first optically detectable signal (i.e. in any case a signal that is different from the first optically detectable signal), is generated in a hydrogel microsphere that is not associated with or does not comprise the analyte. By means of such optically detectable signals, a number of hydrogel microspheres can be determined that are associated with or comprise analyte, on the one hand, and a further number of hydrogel micropsheres can be determined that are not associated with, and do not comprise, analyte, on the other hand. These two sets of hydrogel microspheres with first and second optically detectable signals, respectively, e.g. with and without an optically detectable signal, are herein also sometimes referred to as "positive" hydrogel microspheres" and "negative" hydrogel microspheres", respectively. The total number of detected microspheres is the sum of the positive and negative hydrogel microspheres, i. e. $N = N_{pos} + N_{neg}$. In accordance with the present invention, the concentration $c_s$ of the analyte in the sample may be determined using the formula

$$c_S = \frac{N_B}{V_S} \cdot -ln\left(\frac{N_{neg}}{N}\right) = \frac{N_B}{V_S} \cdot \lambda;$$

wherein,

$c_s$ = concentration of analyte in sample
$N_B$ = pre-defined number of hydrogel microspheres exposed to the sample

$V_S$ = volume of sample containing analyte

$N_{neg}$ = number of detected hydrogel microspheres without an optically detectable signal (= "negative" hydrogel microspheres)

$N$ = number of all detected hydrogel microspheres = $N_{neg} + N_{pos}$.

wherein

$N_{pos}$ = number of detected hydrogel microspheres having an optically detectable signal (="positive" hydrogel microspheres).

Thus, in accordance with the present invention, for a quantification of a concentration of an analyte in a sample, it is no longer necessary to adjust sample concentration or sample volume in any way. As long as the sample volume used is known, the method in accordance with the present invention works with essentially any sample volume. Likewise, for a quantification of a concentration of an analyte in a sample, it is also not necessary to know the volume of the individual reaction compartments, e.g. hydrogel microspheres, as opposed to classical digital amplification methodologies according to the prior art. Rather, it is only necessary to observe how many reaction compartments, e.g. hydrogel microspheres, do or do not have a first optically detectable signal ("positive" and "negative" reaction compartments, e.g. hydrogel microspheres). From such determination of positive and negative reaction compartments, e.g. hydrogel microspheres, it is possible to calculate the total number of detected reaction compartments, e.g. hydrogel microspheres. Putting this into the aforementioned equation together with the pre-defined number $N_B$ of reaction compartments, e.g. hydrogel microspheres exposed to the sample, and the volume of sample that is used in the respective experiment, allows for easy determination of the exact concentration of an analyte in the sample. Thus, a quantitative determination and loss-free analysis of analyte contained in samples with different volumes can be achieved in an easy manner. This is entirely independent of sample volume, and hence the method of quantifying in accordance with the present invention can be used with any sample volume, as long as such sample volume is known.

The entity lambda ("λ"), as can be seen from the aforementioned formula is the negative natural logarithm of the ratio of the negative reaction compartments/hydrogel microspheres to the number of all detected reaction compartments/hydrogel microspheres . Such entity lambda can be interpreted as the mean number of analyte molecules per reaction compartment/hydrogel microsphere.

In accordance with the present invention, said reaction compartments/hydrogel microspheres comprise a material capable of binding said analyte. In this context, binding of analyte to said reaction compartments/hydrogel microspheres may be specific or non-specific. As an example, if the analyte is a nucleic acid, and if the material capable of binding said analyte binds nucleic acids non-specifically, then such material capable of binding said analyte may be a cationic polymer, a cationic oligomer or a cationic monomer or silica. Examples thereof are listed herein.

If, however, material capable of binding said analyte binds such analyte specifically, and the analyte is a nucleic acid, then, such material capable of binding an analyte may for example be an oligonucleotide that is complementary to the nucleic acid of interest, i. e. the target nucleic acid (or target analyte).

If the analyte is a protein, and if said material capable of binding the analyte specifically binds thereto, then such material capable of specifically binding said analyte may typically be an antibody, an antibody fragment or protein receptor that specifically binds to said analyte.

The term "chromogenic enzyme", as used herein, is meant to refer to an enzyme that is capable of converting a chromogenic substrate and thereby generating an optically detectable signal, e.g. a color signal or a fluorescence signal. In order to be able to generate such an optically detectable signal, the chromogenic enzyme typically acts on a chromogenic substrate, which, by the action of the chromogenic enzyme, is processed, e.g. cleaved, and the reaction product of such processing can then be optically detected, for example because it has a particular color or fluorescence that is formed in the course of such processing. Examples of chromogenic enzymes that are frequently used are horseradish peroxidase, calf intestinal alkaline phosphatase, and β-galactosidase. Chromogenic enzymes are capable of acting on chromogenic substrates which, if acted upon by such enzyme, result in accumulation of an optically detectable product, for example a product that is colored or fluorescent, and thus result in the generation of an optically detectable signal. The term "chromogenic enzyme" as used herein, is meant to explicitly also include the possibility that the signal generated thereby, may be a fluorescent signal (generated upon excitation using a particular wavelength (range) rather than only a visually detectable colour signal), and may therefore be detected by a fluorescent channel; in this case, such "chromogenic enzyme" may also be referred to as a "fluorogenic enzyme". Whether or not a visually detectable signal or a fluorescent signal is generated will depend on the choice of the substrate which is acted upon by such enzyme; and such substrate may be "chromogenic" in a strict sense (i.e. generating a visually detectable (coloured) product or signal) or "fluorogenic" (i.e. generating a fluorescent product or signal that may be detected by its fluorescence upon excitation using a particular wavelength (range)).

In embodiments according to the present invention, said reaction compartments/hydrogel microspheres comprise magnetic particles which allow for a mechanical handling and transfer of such reaction compartments/hydrogel microspheres. In one embodiment, such magnetic particles have a size in a range from 50 nm to 10 $\mu$m, preferably 100

nm to 5 μm, more preferably 1 μm to 5 μm, and even more preferably 1 μm to 3 μm.

The term "magnetic particles", as used herein, is meant to referred to particles, showing magnetic behavior, thus allowing such particles to be attracted by a magnet. The magnetic particles used in the context of the present invention are particles that are comprised and/or incorporated into the reaction compartments/hydrogel microspheres according to the present invention. In one embodiment, such magnetic particles are ferromagnetic particles. In another embodiment, such magnetic particles are paramagnetic particles. In yet another embodiment, such magnetic particles are ferrimagnetic particles. In yet another embodiment, such magnetic particles are super-paramagnetic particles. In a preferred embodiment, such magnetic particles are ferromagnetic or paramagnetic particles. The term "magnetic particles" as used herein, is, however, meant to exclude diamagnetic particles. "Magnetic particles", as used herein, preferably have a size and average diameter or an average long extension in one dimension wich allows such magnetic particles to become incorporated into the hydrogel microspheres according to the present invention. Preferably, magnetic particles as used herein, have a size and average diameter or an average long extension in one dimension in a range of from 50 nm to 10μm, preferably 100 nm to 5 μm, more preferably 1 μm to 5μm, even more preferably 1 μm to 3 μm. Preferably the size and average diameter or average long extension in one dimension of the magnetic particles is chosen such that it allows for an incorporation of such magnetic particle(s) into the hydrogel microspheres according to the present invention.

In a preferred embodiment, the reaction compartments/hydrogel microspheres according to the present invention are porous hydrogel microspheres which have an average pore size < 100 nm, preferably < 50nm, more preferable < 25 nm, even more preferably < 15 nm, and even more preferably y 10 nm.

The hydrogel microspheres in accordance with the embodiments of the present invention are herein also sometimes referred to synonymously as "beads" or as "nanoreactor beads" or "NRBs" or "nanoreactors". Furthermore if such hydrogel microspheres additionally comprise magnetic particles (allowing for an easy and simple mechanical handling and transfer of said hydrogel microspheres), they are also sometimes herein referred to as "magnetic hydrogel microspheres" or "magnetic nanoreactor beads" or "mNRBs".

In accordance with embodiments of the method for quantifying a concentration $c_S$ of an analyte in a sample according to the present invention, the reaction compartments/hydrogel micropsheres may be provided in step a) in such a form that they already comprise components for performing an enzymatic amplification reaction such as

(i) a buffer, mono-nucleoside triphosphates an amplification enzyme, a nucleic acid dye for the detection of an amplified nucleic acid, optionally one or more amplification primers or one or more pairs of amplification primers, and further optionally a molecular probe, such as a TaqMan probe or a molecular beacon; or

(ii) a buffer, an analyte-specific antibody or antibody fragment, having a label attached, and a detection reaction for detection for said label; or

(iii) a buffer, an analyte-specific antibody or antibody fragment, a detection antibody for detecting analyte bound to said analyte-specific antibody or antibody fragment, said detection antibody having a label attached, and a detection reagent for detection of said label.

In such embodiment, that is if the reaction compartments/hydrogel microspheres are provided in step a) in such a form that they already comprise components for performing an enzymatic amplification reaction, then they do not need to be subsequently processed by exposing them to a solution for performing said enzymatic amplification reaction, anymore, for example by performing a step b**, as described herein.

In other embodiments, however, these components for performing an enzymatic amplification reaction, may also be only subsequently added in an additional step b**) which is performed before the reaction compartments/hydrogel microspheres are isolated from each other, e.g. transferred into a liquid phase that is immiscible with water an aqueous solution, i.e. before step c).

Embodiments of the present invention may also provide for an additional step b*) which is a washing step, wherein said pre-defined number $N_B$ of reaction compartments/hydrogel microspheres are washed by an appropriate washing solution, for example a washing buffer, by exposing such reaction compartments/hydrogel microspheres to a washing buffer or wash buffer. If such washing step b*) is performed and a processing step b** is necessary or intended to be performed, then the additional step b**) is performed after step b*). Such step b**) involves the processing of said pre-defined number $N_B$ of reaction compartments/hydrogel microspheres by exposing them to a solution which comprises components necessary for performing an enzymatic amplification reaction. For example such solution for performing an enzymatic amplification reaction may comprise:

(i) a buffer, mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; or

(ii) a buffer, an analyte-specific antibody or antibody-fragment having a label attached, and a detection reagent for detection of said label; or

(iii) a buffer, an analyte-specific antibody or antibody fragment, a detection antibody for detecting analyte bound to said analyte-specific antibody or antibody fragment, said detection antibody having a label attached, and a detection reagent for detection of said label;

wherein in (ii) and (iii), said label is either a nucleic acid tag or a chromogenic enzyme; and,

- if said label is a nucleic acid tag, said detection reagent is a solution comprising mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; and,
- if said label is a chromogenic enzyme, said detection reagent is a solution comprising a substrate for said chromogenic enzyme.

In embodiments according to the present invention, in step e) the method involves detection of said first and second optically detectable signals generated in said reaction compartments/hydrogel microspheres, via a first channel, which, preferably, is a fluorescence channel. Because such said first channel allows the detection of positive reaction compartments/hydrogel microspheres and negative reaction compartments/microspheres, it can also be used to determine the total number of all detected reaction compartments/hydrogel microspheres $N$. Alternatively and/or optionally, however, the total number of $N$, may also be determined by optical imaging, via a second channel that is different from said first channel. However, it should be noted that such second channel is not mandatory.

In embodiments according to the present invention, a method of quantifying a concentration $c_s$ of an analyte in a sample, is particularly useful for quantifying a concentration $c_s$ of a target nucleic acid in a sample.

Such method that is specifically tailored for quantifying the concentration of target nucleic acid in a sample comprises the steps:

a) Providing, in any order, an aqueous sample containing, or suspected of containing, a target nucleic acid, and an aqueous suspension of a pre-defined number $N_B$ of reaction compartments/hydrogel microspheres, said reaction compartments/hydrogel microspheres comprising a material capable of non-specifically binding nucleic acids, including said target nucleic acid, or specifically binding said target nucleic acid;

b) allowing said reaction compartments/hydrogel microspheres to take up aqueous sample and to bind said target nucleic acid, preferably all of said target nucleic acid, if present in said aqueous sample, by exposing said pre-defined number $N_B$ of reaction compartments/hydrogel microspheres to said aqueous sample and mixing said aqueous sample with said suspension of reaction compartments/hydrogel microspheres; thus resulting in at least some reaction compartments/hydrogel microspheres of said pre-defined number $N_B$ of reaction compartments/hydrogel microspheres being associated with or comprising said target nucleic acid contained by said aqueous sample provided in step a);

c) transferring said reaction compartments/hydrogel microspheres into a liquid phase that is immiscible with water and aqueous solutions, e. g. an oil-phase, thereby generating a suspension of said pre-defined number $N_B$ of reaction compartments/hydrogel microspheres in said water-immiscible phase, wherein said reaction compartments/hydrogel microspheres are isolated from each other by said water-immiscible liquid phase such that a flow and/or exchange of aqueous sample between different reaction compartments/hydrogel microspheres is not possible and does not occur anymore;

d) performing a nucleic acid amplification protocol on said reaction compartments/hydrogel microspheres that is specific for said target nucleic acid; wherein said nucleic acid amplification protocol results in specific amplification of said target nucleic acid if associated with or comprised by any of said reaction compartments/hydrogel microspheres, and resulting in a generation of a first optically detectable signal in a reaction compartment/hydrogel microsphere, if said reaction compartment/hydrogel microsphere comprises or is associated with amplified target nucleic acid; and wherein said nucleic acid amplification protocol results in no amplification of said target nucleic acid and in a generation of a second optically detectable signal in a reaction compartment/hydrogel microsphere, if said reaction compartment/hydrogel microsphere is not associated with and does not comprise amplified target nucleic acid;

e) determining, by detecting said first and second optically detectable signals, respectively, a number $N_{pos}$ of reaction compartments/hydrogel microspheres having a first optically detectable signal (= "positive" reaction compartment/hydrogel microspheres) and a number $N_{neg}$ of reaction compartments/hydrogel microspheres having a second optically detectable signal (= "negative" reaction compartments/hydrogel microspheres); wherein the total number of detected reaction compartments/microspheres $N = N_{pos} + N_{neg}$;

f) calculating a concentration $c_s$ of a target nucleic acid in said aqueous sample in accordance with formula

$$c_S = \frac{N_B}{V_S} \cdot -ln\left(\frac{N_{neg}}{N}\right) = \frac{N_B}{V_S} \cdot \lambda;$$

wherein

$c_s$ = concentration of target nucleic acid in aqueous sample
$N_B$ = pre-defined number of reaction compartments/hydrogel microspheres exposed to aqueous sample
$V_S$ = volume of aqueous sample containing, or suspected of containing, target nucleic acid
$N_{neg}$ = number of detected reaction compartments/hydrogel microspheres having a second optically detectable signal (= "negative" reaction compartments/hydrogel microspheres)
$N$ = number of all detected reaction compartments/hydrogel microspheres = $N_{neg}$ + $Npos$;
wherein
$N_{pos}$ = number of detected reaction compartments/hydrogel microspheres having a first optically detectable signal (="positive" hydrogel microspheres).

The present inventors have also found that in a method of quantifying a concentration $C_s$ of an analyte in a sample, of particular use is an aqueous suspension of a pre-defined number $N_B$ of reaction compartments/hydrogel microspheres, as defined herein. In embodiments of such aqueous suspension according to the present invention, said pre-defined number $N_B$ of reaction compartments/hydrogel microspheres is in a range of from 1,000 to 1,000,000, preferably in a range of from 5,000 to 500,000, more preferable in a range of from 5,000 to 100,000, even more preferably in a range of from 5,000 to 50,000, and even more preferably in a range of from 5,000 to 20,000.

In order to produce such aqueous suspension of a pre-defined number $N_B$ of reaction compartments/hydrogel microspheres which suspension can be used in a method of quantifying a concentration $c_S$ of an analyte in a sample, the present inventors have also devised a macroparticle which comprises a matrix and a pre-defined number $N_B$ of reaction compartments/hydrogel microspheres which are embedded in such matrix of such macroparticle. The macroparticle according to the present invention can be used to provide the desired number of reaction compartments/hydrogel microspheres, i.e. the aforementioned pre-defined number $N_B$ of reaction compartments/hydrogel microspheres in a convenient manner. For this purpose, the macroparticle in accordance with the present invention is dried, preferably freeze-dried. Effectively, such macroparticle in accordance with the present invention acts as a container or vessel or package for storage and/or transport of a defined number $N_B$ of reaction compartments/hydrogel microparticles. Sometimes, as used herein, the term "macroparticle" is also used synonymously with the term "pellet". In one embodiment, the dimensions of such macroparticle (or "pellet") according to the present invention is chosen such that it has an average diameter in a range of from 1 mm to 50 mm, preferably from 2 mm to 20 mm, more preferably from 2 mm to 15 mm and even more preferably from 2 mm to 10 mm and even more preferably from 2 mm to 5 mm. The macroparticles according to the present invention allow for the simple and straightforward generation of an aqueous suspension of a pre-defined number $N_B$ of reaction compartments/hydrogel microspheres in accordance with the present invention, because they already contain a pre-defined number $N_B$ of reaction compartments/hydrogel microspheres. Such generation of an aqueous suspension of a pre-defined number $N_B$ of reaction compartments/hydrogel microspheres may be performed easily, because of usage of a macroparticle in accordance with the present invention, at a time and place, where such aqueous suspension of a pre-defined number $N_B$ of reaction compartments/hydrogel microspheres is needed or intended. The macroparticles in accordance with the present invention may be stored for extended periods of time and may be used (e.g. for the generation of an aqueous suspension of a pre-defined number $N_B$ of reaction compartments/hydrogel microspheres in accordance with the present invention) at a time and place when the method of quantifying according to the present invention is intended to be performed. In embodiments according to the present invention, the matrix of said macroparticles comprises or is made of a material which is an excipient for drying-processes, in particular for freeze-drying. In one particular embodiment, said excipient for drying, in particular for freeze-drying, is selected from saccharides, such as trehalose, sucrose, mannitol, glucose, fructose, lactose, mannitol, inositol, hydroxypropyl-β-cyclodextrin and combinations thereof; polymers, such as polyethyleneglycol (PEG), polyvinylpyrrolidone (PVP), dextran, gelatin; amino acids, such as arginine, histidine and glycine, and combinations of the foregoing. Such matrix material is preferably chosen such that it allows for a stabilization during the drying process, and yet, at the same time dissolves easily in aqueous solvents or solutions. By appropriate choice of such matrix material, macroparticles in accordance with the present invention may be stored for extended periods of time and used if and when desired.

In accordance with embodiments of the present invention, the macroparticles may be generated by a method comprising the steps:

a) providing, in any order, a suspension of a predefined number $N_B$ of reaction compartments/hydrogel microspheres, as defined herein, an aqueous solvent and at least one matrix material as also defined herein;

b) adding said at least one matrix material to said aqueous solvent, thereby dissolving said matrix material in said aqueous solvent, and mixing said solution of matrix material with said suspension of a predefined number $N_B$ of reaction compartments/hydrogel microspheres, thereby forming a suspension of said reaction compartments/hydrogel microspheres in said aqueous solvent;

c) generating droplets of said suspension formed in step b), such droplets having a defined size and volume, and dispensing such droplets into a water-immiscible liquid phase, e.g. an oil phase that is at a temperature in a range of from -100°C to - 10°C, preferably from -90°C to -20°C, more preferably from -80°C to -30°C; thereby allowing said droplets to freeze;

d) separating said frozen droplets from said water-immiscible liquid phase and drying, preferably freeze-drying them, thus resulting in droplet-shaped macroparticles according to the present invention.

It should be noted that in accordance with embodiments of such method of generating a macroparticle according to the present invention, the matrix material may also or alternatively be provided in step a) together with said aqueous solvent, i.e. as a solution of matrix material dissolved within said aqueous solvent (instead of being provided (e.g. as a solid) separately from said aqueous solvent in step a)); and then, in such embodiments, step b) does not require dissolving matrix material in said aqueous solvent anymore, but merely comprises mixing the aqueous solution of matrix material already provided in step a), with said suspension, preferably with said aqueous suspension, of a predefined number $N_B$ of reaction compartments/hydrogel microspheres, thereby forming a suspension of said reaction compartments/hydrogel microspheres in said aqueous solvent.

In embodiments of a method of generating a macroparticle according to the present invention, in step c), the water-immiscible liquid phase is preferably an oil phase, such as a halogenated hydrocarbon oil, for example a hydrofluoroether (HFE), oil. The advantage of using such halogenated hydrocarbon oil, as opposed to liquid nitrogen or other liquified gases, is that such oil does not evaporate upon contact with droplets of the suspension of hydrogel microspheres in an aqueous solvent. In one embodiment, the water-immiscible liquid phase is an oil phase that is still liquid in a range of from -100°C to -10 °C, preferably from -90°C to - 20°C, more preferably from -80°C to - 30°C. In a particularly preferred embodiment, such water-immiscible liquid phase, e.g. oil phase, has a pour point in a range of from - 50°C to -100°C or even lower. A suitable oil phase that may be used in accordance with embodiments of the present invention is a hydrofluoroether oil available under the tradename NOVEC 7500®.

The term "macroparticle", as used herein, refers to a dried, preferably freeze-dried, particle which comprises a matrix and a pre-defined number $N_B$ of reaction compartments/hydrogel microspheres which are embedded in the matrix of such macroparticle. As opposed to "microspheres" or "hydrogel microspheres" according to the present invention, a "macroparticle" has dimensions in the mm-range, and it can therefore comprise a plurality of "microspheres" or "hydrogel microspheres". Preferably a macroparticle in accordance with embodiments of the present invention has an average diameter in a range of from 1 mm to 50 mm, preferably from 2 mm to 20 mm, more preferably from 2 mm to 20 mm and even more preferably from 2 mm to 10 mm, and even more preferably from 2 mm to 5 mm. In particularly preferred embodiments, such macroparticle is substantially spherical, globular, droplet-shaped, ellipsoidal or otherwise round-shaped. Such "macroparticle" is herein also sometimes referred to as a "pellet".

The term "aqueous solvent", as used herein, refers to water which may optionally comprise additional components dissolved therein, such as a buffer or other solutes.

Further aspects of the present invention are illustrated in and exemplified by the following schemes, figures and examples given merely to illustrate, not to limit the present invention. The scope of protection for the present invention is merely limited by the appended claims.

The terms "of the [present] invention", "in accordance with the invention", "according to the invention" and the like, as used herein, are intended to refer to all aspects and embodiments of the invention described and/or claim herein. As used herein, the term "comprising" is to be construed as encompassing both "including" and "consisting of", both meanings being specifically and explicitly intended, and hence, individually disclosed embodiments in accordance with the present invention. Where used herein, "and/or" is to be taken as a specific disclosure of each of the two specified features or components with or without the other. For example, "A" and/or "B" is to be taken as a specific disclosure of each of (i) A, (ii) B and (iii) A and B, just as if each is set out individually herein. Where an indefinite or definite article is used, wherein referring to a singular noun, e. g. "a", "an" or "the", this includes a plural of that noun unless something else is specifically stated. Likewise, such disclosure equally is meant to be taken as a specific disclosure of a single individual entity initiated by "a", "an" or "the".

BRIEF DESCRIPTION OF THE FIGURES

The present invention is now further described by reference to the following figures, wherein:

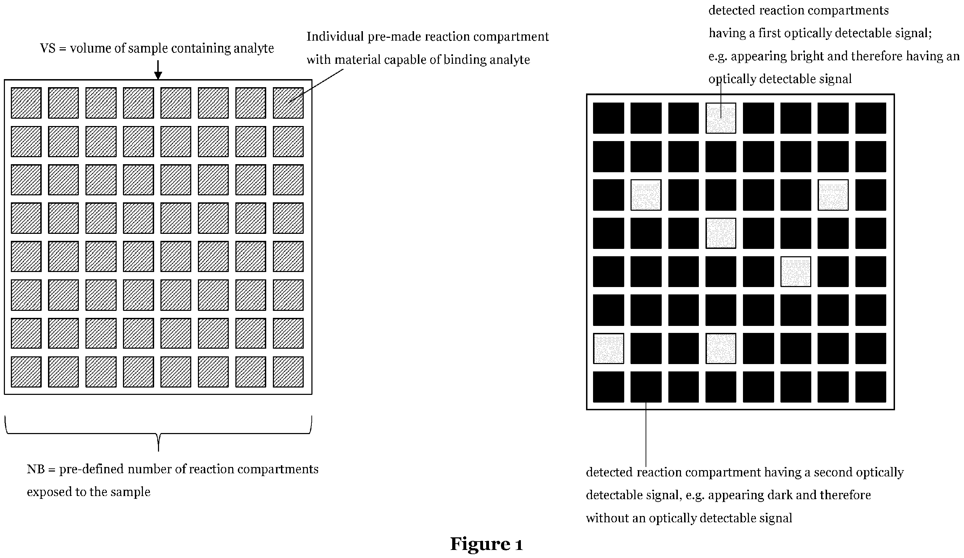

Figure 1 shows a schematic representation of a pre-defined number $N_B$ reaction compartments in accordance with the present invention. Such reaction compartments are entities that comprise a material capable of binding an analyte; and each of such reaction compartments comprise and provide for a volume that is suitable for taking up, and preferably encompassing, an aqueous sample (or a fraction thereof); wherein in such volume of said reaction compartment also an enzymatic or chemical detection reaction, e.g. an enzymatic amplification reaction, for detecting said analyte, can be (subsequently) performed. Also shown is the volume Vs of an aqueous sample containing an analyte, denoted by a square box around the pre-defined number $N_B$ reaction compartments. The left panel shows a pre-defined number $N_B$ of reaction compartments that are exposed to an aqueous sample having a volume $V_S$. In the left panel, the pre-defined number $N_B$ of reaction compartments are allowed to take up aqueous sample and to bind analyte, if present in the aqueous sample, preferably all of the analyte present in the aqueous sample. To that extent, the pre-defined number $N_B$ of reaction compartments are exposed to the aqueous sample in a manner which results in a random distribution of analyte across said pre-defined number $N_B$ of reaction compartments thus resulting in at least some reaction compartments of said pre-defined number $N_B$ of reaction compartments being associated with or comprising the analyte that was contained by the aqueous sample. During such exposure, the pre-defined number $N_B$ of reaction compartments is randomly exposed to the aqueous sample such that distribution of any analyte present in said sample occurs in a random fashion. The right panel shows the pre-defined number $N_B$ reaction compartments after an enzymatic amplification protocol has been performed and a first optically detectable signal has been generated in some reaction compartments which therefore are "positive reaction compartments", and a second optically detectable signal has been generated in some reaction compartments which therefore are "negative reaction compartments". In some embodiments, the generation of a first optically detectable signal in "positive reaction compartments" may be equated with the generation of an optically detectable signal in such "positive reaction compartments", as a result of which such "positive reaction compartments" appear bright (when being detected in an optical detector); and the generation of a second optically detectable signal in "negative reaction compartments" may be equated with "no optically detectable signal" in such "negative reaction compartments", as a result of which such "negative reaction compartments" appear dark (when being detected in an optical detector).

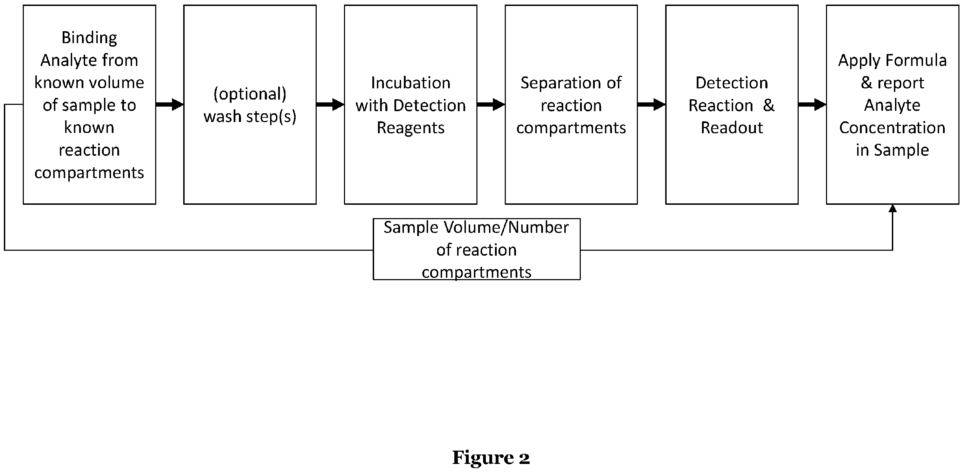

Figure 2 shows a scheme of a generic workflow for the quantification of an analyte in a sample of a known volume with a pre-defined number $N_B$ of reaction compartments capable of binding an analyte (e.g. hydrogel nanoreactor beads). After exposure of a pre-defined number of $N_B$ such reaction compartments to an aqueous sample containing, or suspected of containing an analyte, and one or several optional wash steps, the pre-defined number $N_B$ of reaction compartments are incubated with a solution for performing an enzymatic amplification (e.g PCR master mix), as defined herein, in the figure also referred to as "detection reagents". The reaction compartments are subsequently isolated from each other, an enzymatic amplification reaction is performed, and positive and negative reaction compartments are detected. Thereafter concentration $c_s$ of said analyte in said sample is determined by applying the "calculation formula", as described herein.

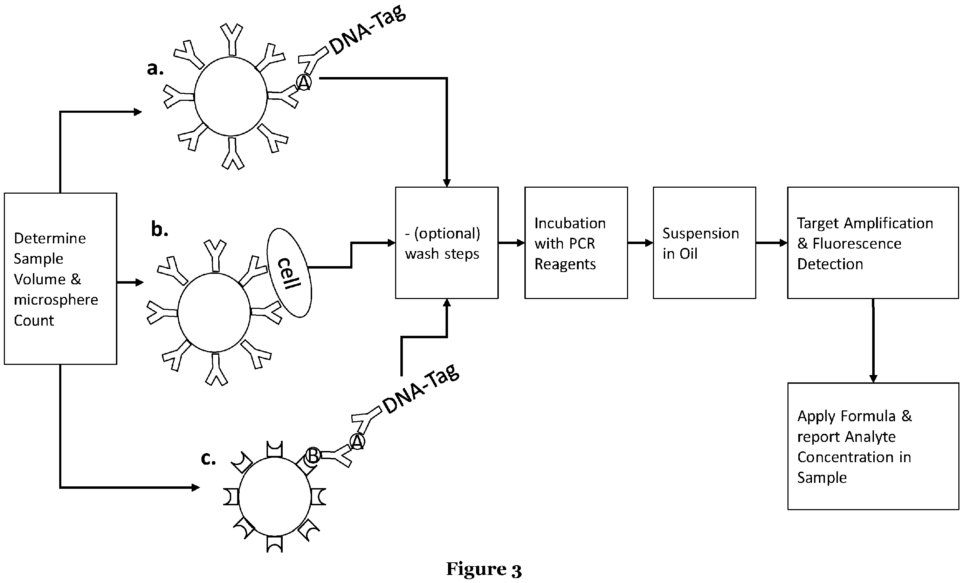

Figure 3 shows a scheme illustrating the workflow for different illustrative forms of antibody-based binding of analytes and subsequent detection of microspheres with bound analyte by nucleic acid amplification using a solution for performing an enzymatic amplification, as defined herein, in the figure also referred to as "PCR reagents".
More specifically, Figure 3a) shows an antibody-coated microsphere that is used to bind analyte (A) and a detection antibody complex, whereby the detection antibody is labeled with a nucleic acid tag. The detection antibody and the antibody on the microsphere, together with the analyte form an analyte-specific immune-complex. For the detection of microspheres with bound analyte, the nucleic acid tag is amplified, for example by "PCR".

Figure 3b) shows an antibody-coated hydrogel microsphere which can be used to capture a biological cell from a sample. Nucleic acid from such cell (DNA or RNA) can be subsequently detected by nucleic acid amplification.

Figure 3c) shows a sandwich (analyte-specific immune-complex) of two analyte-specific antibodies that is formed in solution and that can subsequently be bound to hydrogel microspheres. One of the analyte-specific antibodies is labelled with a nucleic acid tag, the other analyte-specific antibody is labelled with another label (e.g. biotin) that is recognized by a further antibody or receptor attached to said hydrogel microspheres. The hydrogel microspheres are coated with a receptor or antibody specific for the label attached to one of the two antibodies forming the sandwich (e.g. biotin, "B").

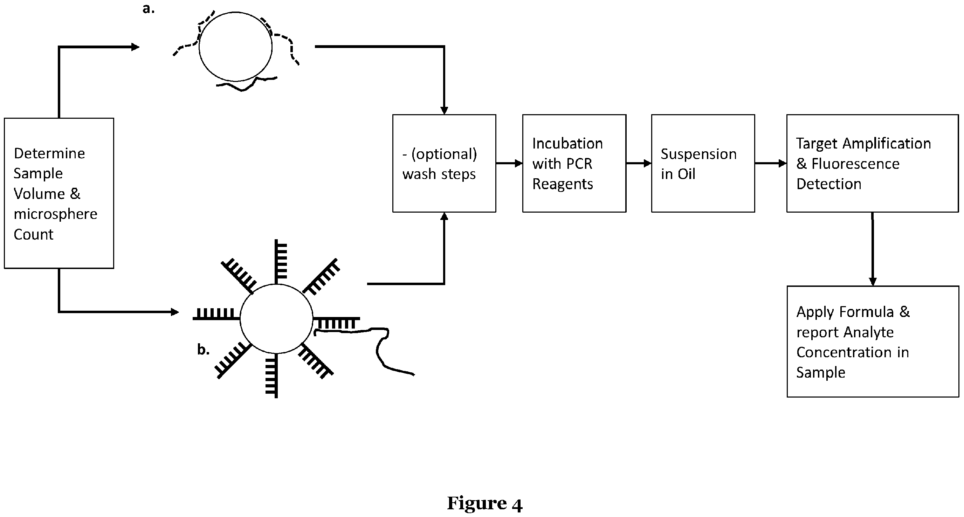

Figure 4 shows a scheme illustrating the workflow for two different forms a nucleic acid binding and subsequent detection of hydrogel microspheres with bound analyte by nucleic acid amplification. More specifically, figure 4a) shows non-specific binding (generic binding) wherein all nucleic acids from a sample are collected and enriched on hydrogel microspheres which are chemically functionalized to bind nucleic acids, for example by charge (e.g. protonated amino groups). In the figure, a target nucleic acid is shown as a solid black line, and other non-target nucleic acids are shown as dotted lines. Figure 4b) shows a specific binding of target nucleic acids, which are enriched on hydrogel microspheres that are equipped with specific complementary binding nucleic acids, e.g. nucleotides.

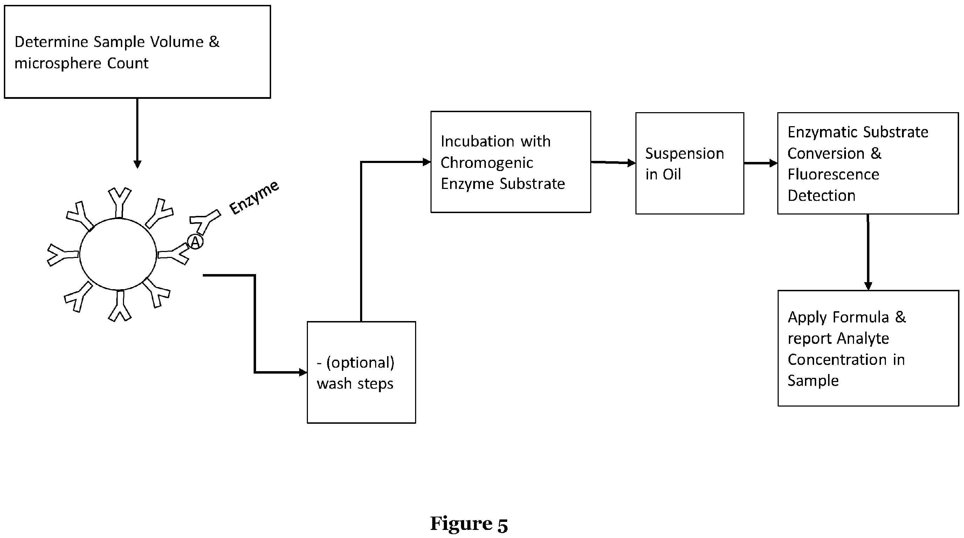

Figure 5 shows a scheme illustrating the workflow for a digital detection of analytes by enzymatic signal amplification following immuno-complex formation on hydrogel microspheres. Instead of a nucleic acid label, here in this exemplary figure, an enzyme label is attached to the detection antibody. Following optional wash steps, the hydrogel microspheres are incubated with a chromogenic enzyme substrate and are suspended in/transferred to an oil phase. Fluorescent product of enzymatic action is accumulated within the space provided by the respective hydrogel microsphere and can be detected.

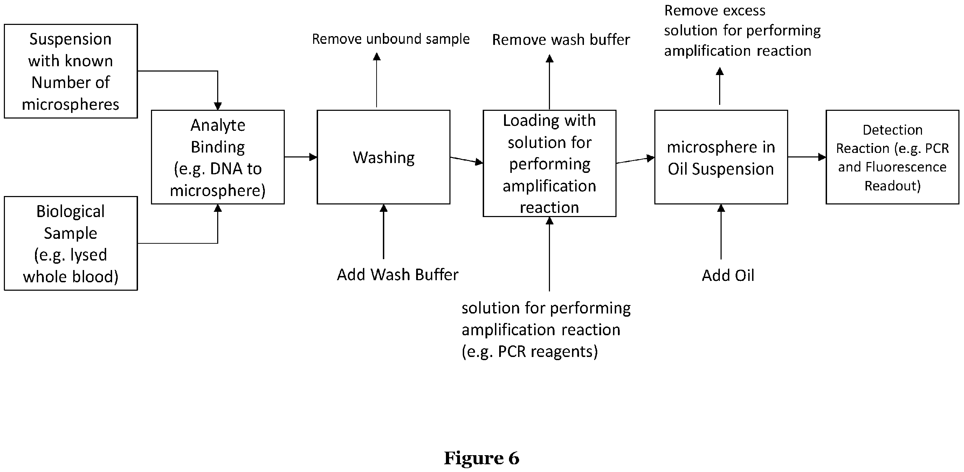

Figure 6 shows a schematic flowchart of an embodiment of the method of quantifying a concentration $c_s$ of a target nucleic acid in a sample;

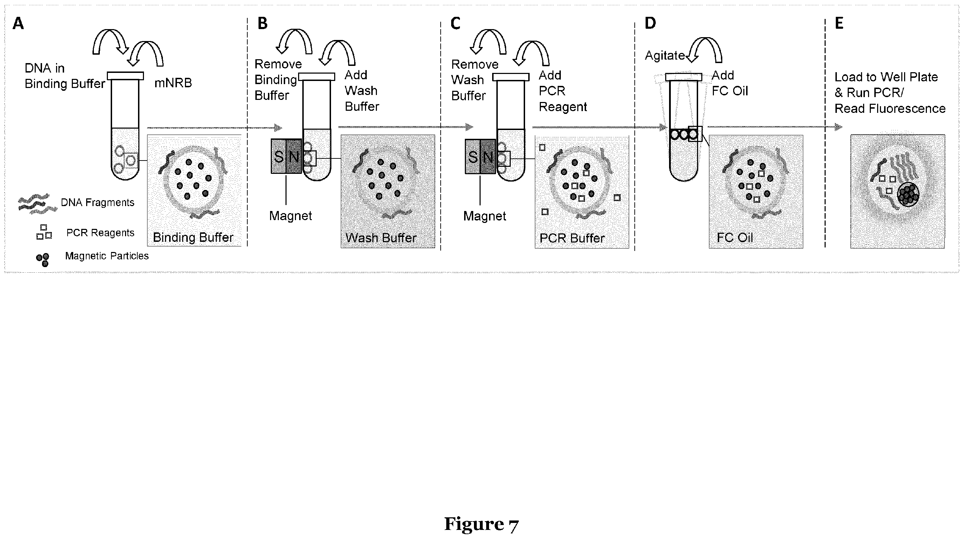

Figure 7 shows a more specific embodiment of a flow-chart of a method quantifying a concentration $c_s$ of a target nucleic acid in a sample;
More specifically, figure 7 A - E shows:

A: Suspension with a known pre-defined number of magnetic hydrogel microspheres is brought into contact with a known volume of sample, comprising purified DNA (e.g. genomic DNA from a cell) in binding buffer; the formed suspension is incubated for 10 minutes at 40°C on thermomixer at 2.000 rpm
B: the tube with the suspension is put on a magnetic stand, the binding buffer is removed and replaced with a wash buffer, the tube is vortexed at room temperature
C: the tube is placed on a magnetic rack and the wash buffer is removed from the hydrogel microspheres, PCR mastermix is added to the microspheres, the tube is briefly vortexed and the mastermix solution is allowed to equilibrate with the wash buffer remaining within and around the hydrogel microspheres
D: oil is added to the tube; the tube is subjected to strong agitation (e.g. on a vertical mixer) allowing to form a water in oil emulsion and a microsphere in oil suspension
E: the suspension is loaded on a well plate or transferred into a PCR tube and subjected to thermocycling conditions; following PCR the fluorescence signal for each hydrogel microsphere is detected

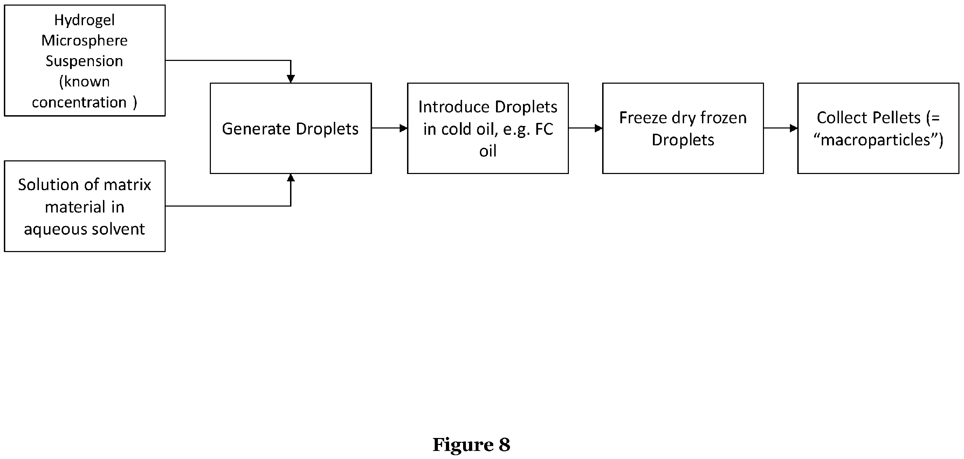

Figure 8 shows a schematic representation of an embodiment of a method of generating a macroparticle ("pellet") in accordance with the present invention; From a mixture of an aqueous suspension of hydrogel microspheres and a solution of matrix material in an aqueous solvent droplets are generated which are introduced into a cold oil phase which allows to freeze said droplets. These can be subsequently freeze-dried and collected as macroparticles in accordance with the present invention ("pellets").

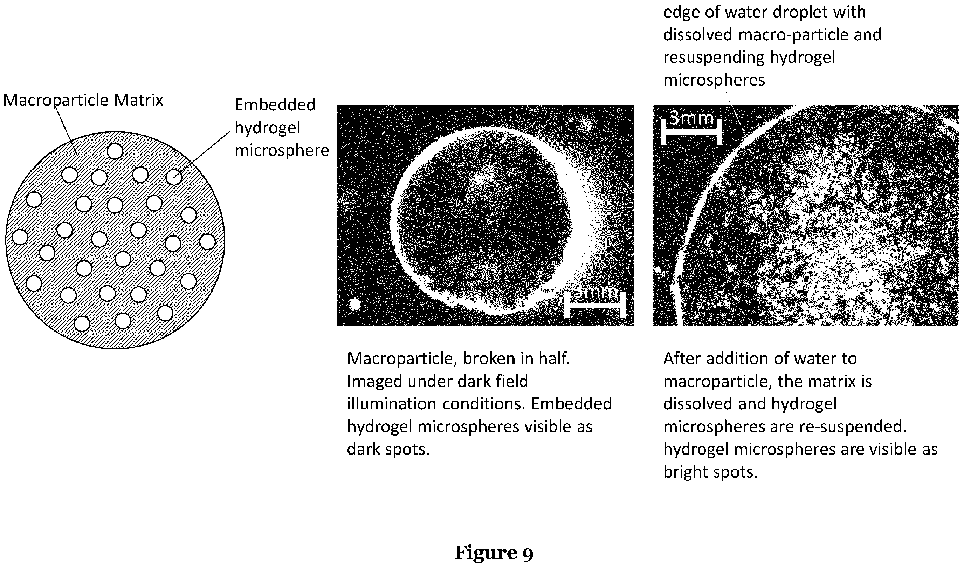

Figure 9 shows an embodiment of a schematic representation of a macroparticle in accordance with the present invention; a scale bar in the photographs illustrate the dimensions of the macroparticle

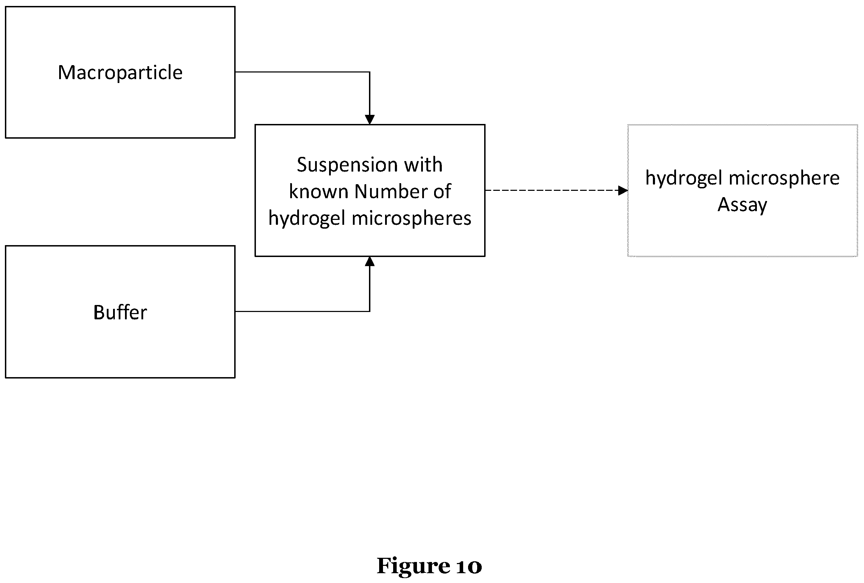

Figure 10 shows a schematic representation of an embodiment of a flow-chart of a method for generating a suspension of a pre-defined number $N_B$ of hydrogel microspheres; this can be subsequently used in a method of quantifying a concentration $c_s$ of an analyte in a sample in accordance with embodiments of the present invention ("hydrogel microsphere assay").

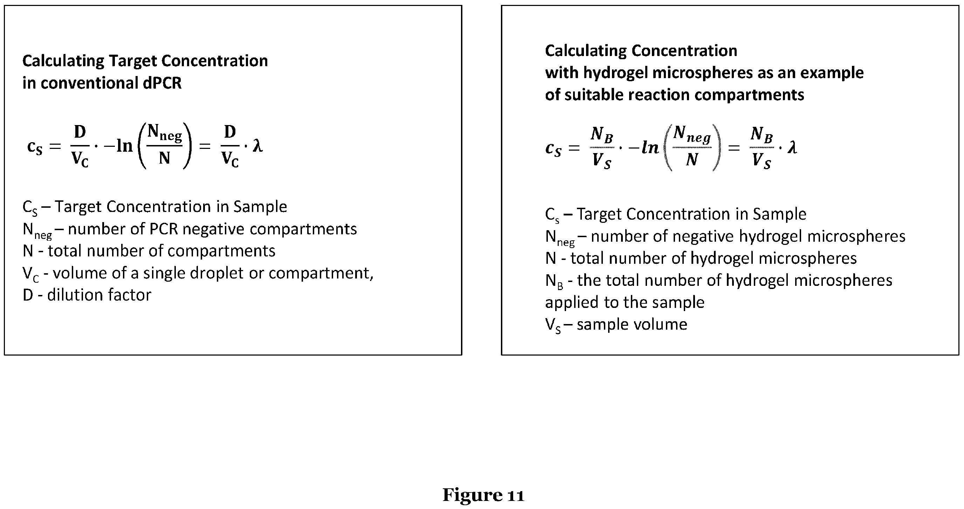

Figure 11 shows the formula for calculating a concentration of a target analyte in accordance with embodiments of the method of the present invention (right-hand box) versus the formula that is conventionally used in dPCR (left-hand box).

Figure 12 shows plots of fluorescence intensity and corresponding images of magnetic hydrogel microspheres ("magnetic nanoreactor beads" = "mNRBs") of experiments as described in Example 3. Data are shown for five sample dilution levels ($V_1$-$V_5$) and illustrative fluorescence images of magnetic hydrogel microspheres ("magnetic nanoreactor beads" = "mNRBs") at different magnifications.

Figure 13 shows results of experiments using magnetic hydrogel microspheres ("magnetic nanoreactor beads" = "mNRBs") as described in Example 3.

Figure 14 shows calculated sample concentrations for each individual measurement (12 replicates) plotted against the expected concentration values for the experiments described in Example 3.

Moreover, in the following, reference is made to the examples, which are given to illustrate, not to limit the present invention.

## EXAMPLES

### Example 1:

Example in which freeze dried macroparticles ("pellets") with embedded hydrogel microspheres ("nanoreactor beads") are produced for downstream processing.

First a suspension of nanoreactor beads in a medium suitable for freeze-dying is prepared. Such a suspension typically consists of 15-30% trehalose and 1.5%-5% PEG 6000 or other suitable lyo-protectant such as for example Cavasol or Sucrose , and the nanoreactor beads. The suspension is produced by adding an appropriate volume of the excipients containing solution at an appropriately adjusted concentration to a volume of liquid containing a known number of magnetic nanoreactor beads as described previously in patent application EP 21 206 745.8 (e.g. 1000 nanoreactor beads per $\mu$L). The suspension is incubated at room temperature while moderately shaking at room temperature for approx. 30 minutes to allow for equilibration of the different liquids.

For macroparticle ("pellet") production, individual droplets of the previously prepared suspension are dispensed into cold hydrofluoroether (HFE) oil (e.g. Novec 7500, 3M). For this purpose, the suspension, which tends to sediment, is kept homogeneous by continuous stirring. Droplet formation can be accomplished in a variety of ways, for example, with commercially available dispensers, with pipettes, syringe pumps, or other droplet generators. The HFE oil used can be cooled (e.g. with dry ice) to a temperature that is below -60°C but above the pour point of the oil. For Novec 7500, this is at -110°C. Preferably, a sieve is inserted into the oil, with which the droplets can be removed after freezing and transferred to a lyophilization instrument.

The frozen droplets with a total volume of 20$\mu$L and containing 10.000 nanoreactor beads each are lifted from the HFE oil with the cooled sieve and transferred to a lyophilization instrument where they are dried, e.g., at 0.2 mbar and -70°C for 4 hours plus 1 hour of post-drying at 22°C.

Thereafter the dried macroparticles ("pellets") comprising for instance 10.000 nanoreactor beads, are collected in sealed clean glass vials for storage.

### Example 2:

Re-constitution of nanoreactor beads from freeze-dried macroparticles ("pellets")

A single macroparticle ("pellet") is applied to a 2ml Eppendorf tube. 200$\mu$L of distilled water are added to the macroparticle ("pellet"). The tube is closed and put on a thermomixer instrument. The tube is shaken for 15 minutes at 40°C at 2.000 rpm. Thereafter the tube is transferred to a magnetic stand and the supernatant removed. Alternatively with nonmagnetic nanoreactor beads centrifugation or filtration could be used to remove the supernatant. 20 $\mu$L of binding buffer (50 mM sodium malonate, pH 2.8 and 0.1% PEG 6000) are added to the beads remaining in the tube. The tube is briefly vortexed and the suspension of 20$\mu$L containing 10.000 beads is now ready for the binding step with the nucleic acid containing sample. Such reconstituted beads from freeze-dried macroparticles ("pellets") show the same performance as non-freeze-dried beads in digital PCR assays.

### Example 3:

Use of pre-counted nanoreactor beads (i.e. with a pre-defined number $N_B$) for precise concentration measurements

Such re-constituted magnetic nanoreactor beads have been processed for digital PCR on the BLINK X Product platform (BLINK, Jena, DE). An assay for quantification of the RPP30 target, a single copy gene in human DNA, was used. We have detected the target in human DNA containing a sequence target from the RPP30.

Primers and a probe specific for human RPP30 gene (forward primer sequence (5'-3'): GCC AAA TTC TGC TCG TTG TTA G (SEQ ID NO:1); reverse primer sequence (5'-3'): CTT CCC TCA CGG CAT ATA CTT C (SEQ ID NO:2); probe sequence (5'-3'): Atto488- TCA CCA GCT GGA TGT CCA CAT TCA-BHQ-1) (SEQ ID NO:3) were used to quantify human genomic DNA. Final concentration for primers and probe was 0.9 $\mu$M and 0.25 $\mu$m, respectively.

DNA was isolated from human Buffy Coat by using the Flexi-Gene DNA Kit (Qiagen) according to the manufacturer's instructions and stored at -20°C until used. On the day of the experiment, the DNA was thawed, and the concentration of the material stock determined photometrically. A serial 10-fold dilution was prepared over 5 steps providing DNA samples that subsequently underwent the sample processing protocol with hydrogel microspheres ("nanoreactor beads").

For DNA binding, reconstituted magnetic hydrogel microspheres, i.e. hydrogel microspheres comprising magnetic particles ("magnetic nanoreactor beads" or "mNRBs") were incubated with a solution containing target DNA in binding buffer (50 mM sodium malonate, pH 2.8 and 0.1% PEG 6000) for 10 min at 30°C at 2000 rpm on a thermomixer instrument (Eppendorf). The supernatant was removed from the mNRBs after placing the tube on a magnetic stand and followed by washing with 200µl of wash buffer (12.5 mM sodium malonate, 0.001% tergitol). Wash buffer was removed after placing the beads on the magnetic stand and mNRBs were loaded with PCR reagents. The concentration of the PCR reagents in the final mix was 100 mM Tris-Aminomethan, 22 mM potassium chloride, 22 mM ammonium chloride, 3 mM magnesium chloride, 0.2 U/µl Hot Start Taq DNA Polymerase (biotechrabbit), 0.2 mM dNTPs (biotechrabbit), 0.1% (w/v) BSA (Sigma), 0.9 µm primers and 0.25 µM probe (Metabion). Beads were incubated with PCR reagents for 5 min on a Thermomixer (Eppendorf) at 30°C at 1800 rpm. Next, supernatant was removed from mNRBs on the magnetic rack. For mNRB emulsification, fluorocarbon oil with 5% Pico-Surf (Sphere Fluidics) was added, and agitation on a Minilys Homogenizer (Bertin Technologies) was applied 3 times for 5 seconds at level 2. Excess oil was removed, and new oil was added to mNRBs three times to remove microemulsion from the previous emulsification step. 3.5 µl of emulsified NRBs were transferred into each well on a Blink X mini-well plate (Blink AG).

For amplification and detection, the BLINK X (BLINK AG) instrument was used. The instrument features a thermocycling module equipped with a Peltier element and a fluorescence imaging module providing for four channels for fluorescence detection. The system is designed to process mini-plates made of plastics with high thermal conductivity. The plate is positioned on a dedicated magnetic loading rack for filling and aligning mNRB suspensions in a self-assembled monolayer, and filled with 960 µl FC oil containing 0.5% Picosurf. To each of the 6 wells of the plate (120 µm depth, 7 mm diameter), the respective reaction-ready emulsified mNRBs are pipetted. The mNRBs are pulled to the bottom of the wells by the magnetic force of the rack. A cover slip is placed into the mini-plate, thereby isolating wells from each other. The mini-plate is closed with a transparent lid containing a gasket at its rim consisting of thermoplastic elastomer to allow proper melt sealing upon subsequent heat exposure. The closed mini-plate is transferred to the thermocycling module where it is tightly pressed onto a 40 $\times$ 40 mm Peltier element for optimal thermal coupling. The module is inserted in the Blink X instrument and thermo-cycled according to the following temperature profile: initial system priming for 120 s at 80°C, initial denaturation at 94°C for 60 s followed by 45 cycles of 94°C for 2 s, 62°C for 2 s. Endpoint fluorescence imaging is performed at 30°C. The mNRBs are imaged on the mini-plate still located in the thermocycling module with the Blink X instrument. For each well position on the mini-plate, 4 images are acquired in each detection channel. Acquired images are saved as raw images and as stitched overview images of the entire plate in bmp format. Data analysis on the BLINK X platform comprises two subsequent analysis steps: image analysis and digital PCR analysis. Image analysis with the image segmentation algorithm utilizes the concept of maximally stable extremal regions to recognize beads. Based on the identified regions with increased fluorescence signal intensities, circles are fitted that define the outer boundaries of the beads. The representative fluorescence signal intensity of each bead is derived from a fluorescence model assuming that the beads are spherical. Bead size is directly calculated from the enclosing circles representing outer bead boundaries. Shape and size information are considered for further data processing to detect optical artifacts and apply valid/invalid criteria for each detected NRB. Digital PCR analysis evaluates estimated fluorescence signals of beads in the detection channel. As a first step, a fluorescence intensity threshold is determined that distinguishes PCR positive and negative beads. Here, modality of the fluorescence intensity distribution is determined by fitting a one-component and a two-component Gaussian mixture model (GMM) to the data and selecting the preferred model by comparing their Bayesian information criterion (BIC) scores. In case of a bimodal distribution, the intensity threshold is the position of the local minimum between the GMM mean values. A Kernel density estimate is applied for curve smoothing and determination of the local minimum. After threshold setting, Poisson statistics evaluates the targets per bead based on the proportion of PCR negative beads $N_{neg}$ in the total quantity of beads N. The final quantitation of the amount of target in the sample Cs is calculated according to the following equation

$$c_S = \frac{N_B}{V_S} \cdot -ln\left(\frac{N_{neg}}{N}\right) = \frac{N_B}{V_S} \cdot \lambda$$

Here the mean number of targets per bead (A) is determined by calculating the negative natural logarithm of the quotient from the number of detected negative beads $N_{neg}$ and the number of all detected beads N. By multiplying A with the quotient of the total number of beads applied or exposed to the sample $N_B$ and the volume of the sample containing the targets $V_S$ the concentration of targets in a sample (Cs) is calculated.

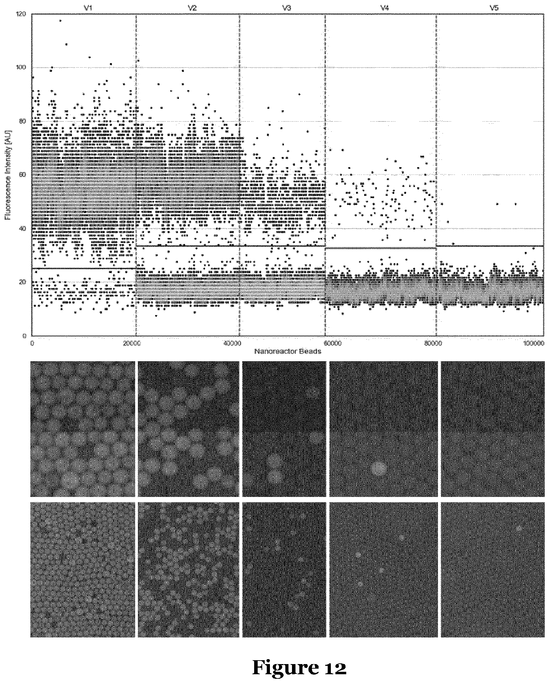

Collected fluorescence data for all thermocycled beads and exemplary images with enlarged sections, are shown in Figure 12. In Figure 12 data are shown for five sample dilution levels (V1 - V5) and illustrative fluorescence images of the hydrogel microspheres at different magnifications.

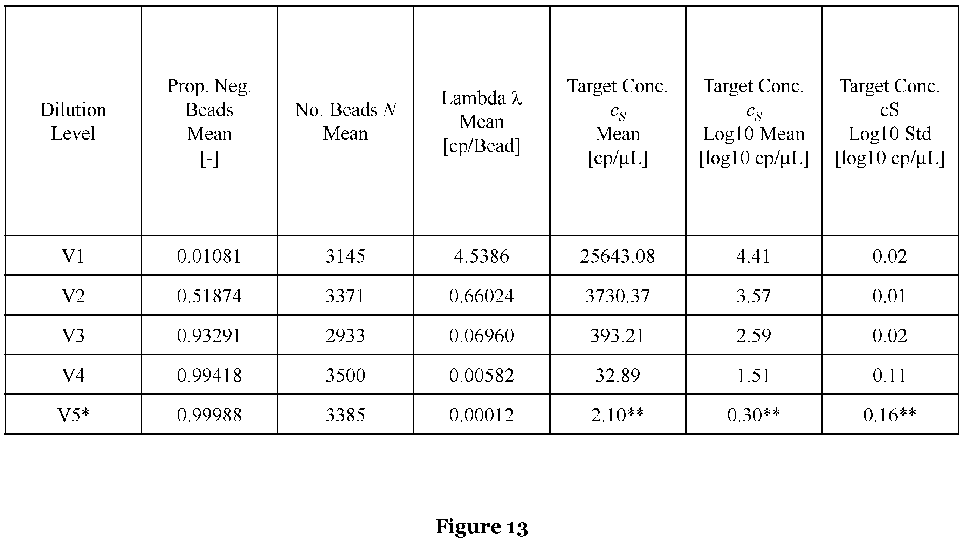

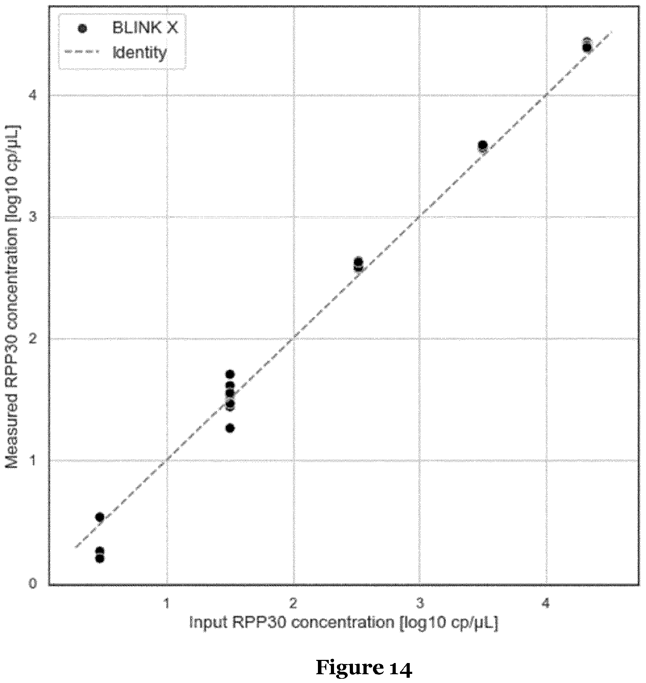

The obtained results have been applied to the formula above for each sample. The calculated results are summarized in the table shown in figure 13 and graphically represented in figure 14. The table shows the dilution levels of samples used ("Dilution Level"), the mean proportion of negative beads to detected beads ("Prop. Neg. Beads Mean") ($N_{neg}$/N), the mean number of beads used in the measurement ("No. Beads $N$ Mean"), the mean value of lambda ("Lambda $\lambda$ Mean"), the mean target concentration $c_S$ ("Target Conc. $c_S$ Mean", measured in copies per microliter, "cp/$\mu$l")), the average of the decadic logarithms of target concentrations $c_S$ ("Target Conc. $c_S$ Logic Mean"), and the standard deviation of the decadic logarithms of the target concentrations $c_S$ ("Target Conc. $c_S$ Logic Std").

In this specific experiment a pre-defined number $N_B$ of hydrogel microspheres = 90,400 was used. For the highly diluted sample V5 sensitivity is somewhat reduced. In this case, only four of twelve replicates provide a non-zero result. This is because not all of the 90.400 mNRB incubated with and exposed to the respective sample dilution are ultimately transferred to the mini-plate wells for PCR and imaging. The mean number of beads per well (replicate) used here is approximately 3300. The total number of analyzed beads per dilution level is therefore 39.600, which is 44% of all mNRBs applied. Assuming a true V5 sample RPP30 concentration of 3.18 cp/pL, the expected number of targets per test replicate with 3300 beads is only 1.9. Based on Poisson distribution, in this case detection rate cannot be better than 85%. Nevertheless the method works over an extremely wide range of analyte concentrations and even with highly diluted samples. Precision of the technical replicates is measured as standard deviation of log10 transformed target concentrations. Overall, the system behaves in excellent agreement with the statistically expected values.

Lambda values obtained with mNRBs are directly derived from the proportion of PCR negative beads using Poisson statistics. As outlined herein, the entity lambda ("$\lambda$"), as can also be deduced from the aforementioned calculation formula herein, is the negative natural logarithm of the ratio of negative hydrogel microspheres to the number of all detected hydrogel microspheres:

$$-ln\left(\frac{N_{neg}}{N}\right)$$

Such entity lambda can be interpreted as the mean number of analyte molecules per hydrogel microsphere ("copies per bead" or "cp/bead"). Therefore, target concentrations are determined according to the developed equation in accordance with the present invention. Each sample was incubated with a pre-defined total number $N_B$ of 90,400 mNRBs. The sample volume $V_S$ for each dilution is 16 $\mu$L. Due to the significant binding capacity of the mNRBs, complete target capture can be assumed, and therefore no adjustments are made for potential target loss during washing and master mix loading. Accordingly, the calculation was performed as follows:

$$c_S = ((565 \text{ beads}/(\mu L) \times 160 \ \mu L)/(16 \ \mu L)) \times \lambda = ((90400 \text{ beads})/(16 \ \mu L)) \times \lambda$$

In Figure 14, the obtained concentration values for each individual measurement are plotted against the expected concentration demonstrating an excellent agreement between expected values and actually obtained values using the calculation in accordance with the present invention. In the figure, for each dilution 12 replicates are plotted with the exception of the lowest dilution (V5 of Figures 12 and 13) where only 4 replicates are plotted.

**Example** 4:

**Embodiment: digital enzyme immuno assay**

The methodology according to embodiments of the present invention is also applicable for quantitating any soluble analyte by some form of immunoassay carried out on the described hydrogel micropsheres ("nanoreactor beads") and by applying the invented formula for quantification of analyte in the sample. Such assays may for instance fall into the well-known category of immuno-PCR ([1,2]). Others have also previously used conventional droplet volume based digital PCR readout for quantitation in such immuno-PCR assays [3]. However, none of these have employed a methodology which is independent of sample volume and for which the volume of the amplification compartments does not need to be known.

In this particular embodiment the present inventors describe a process of establishing a digital immunoassay for the detection of human cTnI in combination with the newly invented quantification approach. This is also schematically shown in figure 3c). A DNA-labeled detection antibody and a biotin-labeled capture antibody form a sandwich complex with the antigen in solution. This complex is trapped on a known number of streptavidin-coated hydrogel microspheres ("nanoreactor beads"). Unbound detection antibody, and thus the DNA label, is removed by appropriate washing steps. Following incubation with a solution with reagents for carrying out a PCR amplification and detection, the nanoreactor beads are suspended in oil so that separate reaction compartments are formed. The nanoreactor beads are loaded on a Blink X (BLINK AG) well-plate, as described in European patent application no. EP22165559.0. The well plate is transferred to a Blink X instrument for thermocycling, fluorescence detection and data analysis. The concentration of analyte in the sample is determined by applying the process described in the previous embodiments and the present patent application.

Detection Antibody

The cTnI detection antibody (clone 3H9, SDIX) is labeled using the Thunder-Link® PLUS Oligo Conjugation System (Innova Bioscience) according to the manufacturer's protocol and then purified. The following sequence is coupled to the antibodies:

5'GCGTCAGACCCCGTAGAAAAGATCAAAGGATCTTCTTGAGATCCTTTTTTTCTGCGCGTAA

TCTGCTGCTTGCAAACAAAAAAACCACCGCTACCAGCGGTGGTTTGTTTGCCGGATCAAGAG

CT3'

(SEQ ID NO:4)

Capture Antibody:

Clone TPC-110 (SDIX) is used as a capture antibody. This was labelled with Biotin by Lightning link Streptavidin (Innova Bioscience) according to the manufacturer's protocol.

Preparation of hydrogel microspheres ("nanoreactor beads"):

A suspension containing a defined number of nanoreactor beads (e.g. 21.000 beads in a volume of 40μl) in PBS buffer is mixed with 5μL of a solution containing streptavidin (1mg/mL) in PBS and incubated for 15 minutes at 37°C on a thermomixer (Eppendorf) at 2000 rpm. Thereafter the tube is transferred to a magnetic stand (BLINK X processing rack, Blink AG) and the supernatant replaced with 200μL of pure PBS. The procedure is repeated two times.

Forming of the immune complex and its capture

The following reaction mix is applied.

| | |
|---|---|
| human plasma | 80μl |
| TBS(K) pH 8,4 (20mM Tris, 50mM KCl pH 8,4), 0,5% TritonX-100, 10mg/ml BS | 10μl |
| HBR-Plus (Scantibodies) | 10μl |
| DNA labelled detection antibody | x μl |
| Streptavidin labelled capture antibody | y μl |

Optimization of antibody concentration:

Optimal concentration of detection and capture antibodies is determined by conventional immuno-PCR. The concentrations of the two antibodies were systematically varied and immuno-complexes using Troponin-free plasma (negative controls) and troponin-free plasma with defined amounts of spiked Troponin I generated. These were captured on particles, washed and subjected to conventional PCR. Optimum concentration of the respective antibodies is indicated by the lowest limit of detection and broadest dynamic measurement range.

Generating and capturing the immune-complex:

100μl of reaction mixture (see above) is prepared with the previously determined optimum concentrations of the two antibodies. The reaction mixture is incubated for 10 min at 37°C at 2000rpm on an Eppendorf thermomixer.
In parallel pre-counted nanoreactor beads in PBS are placed on a magentic rack and the supernatant removed. The reaction mixture is added to the beads. Following a brief vortexing step the suspension is incubated on a thermomixer for 5 min at 25°C at 800rpm. During this time the binding of the streptavidin-labeled capture antibodies including the immune complexes to the nanoreactors is accomplished.
Subsequently 5 washing steps are performed with 500 μl of TBS (K) pH 8.4 (20 mM Tris, 50 mM KCl pH 8.4), 0.05% TritonX-100, 1 mg / ml BSA suing a magnetic rack and vortexer.

Downstream processing:

After removing the wash buffer 50µL of PCR reaction mixture is added to the nanoreactors.
The mixture has the following composition:

500nM fw-Primer (5' AGCTCTTGATCCGGCAAACA 3') (SEQ ID NO:5)
500nM rev-Primer (5' GCGTCAGACCCCGTAGAAAA 3') (SEQ ID NO:6)
SYBR® Green I nucleic acid gel stain (Sigma-Aldrich, #S9430) 1:25000
2 units TaqPolymerase
2× PCR Buffer ([40 mM Tris HCl (pH 8.4), 100 mM KCl]
PCR grade Water

The suspension is incubated with PCR reagents for 5 min on a Thermomixer (Eppendorf) at 30°C at 1800 rpm. Next, supernatant is removed after placing the suspension in a tube on a magnetic rack. For oil suspension, fluorocarbon oil with 5% Pico-Surf (Sphere Fluidics) was added, and the tube was agitated on a Minilys Homogenizer (Bertin Technologies) 3 times for 5 seconds at level 2. Excess oil was removed, and new oil was added to the nanoreactors three times to remove any microemulsion. 3.5 µl of emulsified NRBs were transferred into 6 wells of a Blink X mini-well plate, as described in European patent application no. EP22165559.0. PCR, fluorescence detection is performed according to the protocol from the previous examples and embodiments.
It is clear that other forms of digital immunoassays may be performed with the present inventors' approach for analyte quantification. For instance, a format like SiMoA (single molecule array)[4,5] may easily be adapted to the present inventors' approach, by for instance binding a first antibody to a nanoreactor bead and coupling a second antibody to a reporter enzyme (see also figure 5)). After forming the immuno-sandwich on the nanoreactor beads and exposing the suspension to a fluorogenic enzyme substrate the beads would transferred to oil and incubated for fluorescence imaging. By applying the invented formula precise quantification of analyte in a solution can be obtained.

[1] M. Spengler, M. Adler, C.M. Niemeyer, Highly sensitive ligand-binding assays in pre-clinical and clinical applications: immuno-PCR and other emerging techniques, Analyst. 140 (2015) 6175-6194. https://doi.ors/10.1039/c5an00822k.

[2] L. Chang, J. Li, L. Wang, Immuno-PCR: An ultrasensitive immunoassay for biomolecular detection, Anal Chim Acta. 910 (2016) 12-24. https://doi.org/10.1016/j.aca.2015.12.039.

[3] H. Schröder, M. Grösche, M. Adler, M. Spengler, C.M. Niemeyer, Immuno-PCR with digital readout, Biochem Bioph Res Co. 488 (2017) 311-315. https://doi.org/10.1016/j.bbrc.2017.04.162.

[4] D.M. Rissin, C.W. Kan, T.G. Campbell, S.C. Howes, D.R. Fournier, L. Song, T. Piech, P.P. Patel, L. Chang, A.J. Rivnak, E.P. Ferrell, J.D. Randall, G.K. Provuncher, D.R. Walt, D.C. Duffy, Single-Molecule enzyme-linked immunosorbent assay detects serum proteins at subfemtomolar concentrations, Nat Biotechnol. 28 (2010) 595-599. https://doi.org/10.1038/nbt.1641.

[5] S.M. Schubert, L.M. Arendt, W. Zhou, S. Baig, S.R. Walter, R.J. Buchsbaum, C. Kuperwasser, D.R. Walt, Ultrasensitive protein detection via Single Molecule Arrays towards early stage cancer monitoring, Sci Rep-Uk. 5 (2015) 11034. https://doi.org/10.1038/srep11034.

The features of the present invention disclosed in the specification, the claims, and/or in the accompanying figures may, both separately and in any combination thereof, be material for realizing the invention in various forms thereof.

Claims

1.    A method of quantifying a concentration $c_s$ of an analyte in a sample, said method comprising the steps:

a) providing, in any order, an aqueous sample containing, or suspected of containing, an analyte, and a predefined number $N_B$ of reaction compartments, said reaction compartments comprising a material capable of binding said analyte;

b) allowing said reaction compartments to take up aqueous sample and to bind said analyte, preferably all of said analyte, if present in said aqueous sample, by exposing said pre-defined number $N_B$ of reaction compartments to said aqueous sample in a manner which results in a random distribution of analyte across said pre-defined number $N_B$ of reaction compartments, thus resulting in at least some reaction compartments of said pre-defined number $N_B$ of reaction compartments being associated with or comprising said analyte contained by said aqueous sample provided in step a);

c) optionally, if a flow and/or exchange of aqueous sample between different reaction compartments resulting from step b) is still possible and/or occurs after step b): isolating said reaction compartments resulting from step b) from each other by separating them from each other such that a flow and/or exchange of aqueous sample between different reaction compartments is not possible and does not occur anymore;

d) performing an enzymatic amplification protocol within said reaction compartments, wherein said enzymatic amplification protocol generates a first optically detectable signal in a reaction compartment being associated with or comprising said analyte; and wherein said enzymatic amplification protocol generates a second optically detectable signal in a reaction compartment not being associated with or not comprising said analyte;

e) determining, by detecting said first and second optically detectable signals, respectively, a number $N_{pos}$ of reaction compartments having a first optically detectable signal (= "positive" reaction compartments) and a number $N_{neg}$ of reaction compartments having a second optically detectable signal (= "negative" reaction compartments); wherein the total number of detected reaction compartments $N = N_{pos} + N_{neg}$;

f) calculating a concentration $c_s$ of said analyte in said aqueous sample in accordance with formula

$$c_S = \frac{N_B}{V_S} \cdot -ln\left(\frac{N_{neg}}{N}\right) = \frac{N_B}{V_S} \cdot \lambda;$$

wherein

$c_s$ = concentration of analyte in aqueous sample

$N_B$ = pre-defined number of reaction compartments exposed to aqueous sample

$V_S$ = volume of aqueous sample containing, or suspected of containing, analyte

$N_{neg}$ = number of detected reaction compartments having a second optically detectable signal (= "negative" reaction compartments)

$N$ = number of all detected reaction compartments = $N_{neg} + N_{pos}$.

wherein

$N_{pos}$ = number of detected reaction compartments having a first optically detectable signal (="positive" reaction compartments).

2. A method of quantifying a concentration $c_s$ of an analyte in a sample, in particular according to claim 1, said method comprising the steps:

a) providing, in any order, an aqueous sample containing, or suspected of containing, an analyte, and an aqueous suspension of a pre-defined number $N_B$ of hydrogel microspheres, said hydrogel microspheres comprising a material capable of binding said analyte;

b) allowing said hydrogel microspheres to take up aqueous sample and to bind said analyte, preferably all of said analyte, if present in said aqueous sample, by exposing said pre-defined number $N_B$ of hydrogel microspheres to said aqueous sample and mixing said aqueous sample with said suspension of hydrogel microspheres; thus resulting in at least some hydrogel microspheres of said pre-defined number $N_B$ of hydrogel microspheres being associated with or comprising said analyte, contained by said aqueous sample provided in step a);

c) transferring said hydrogel microspheres into a liquid phase that is immiscible with water and aqueous solutions, e.g. an oil-phase, thereby generating a suspension of said pre-defined number $N_B$ of hydrogel microspheres in said water-immiscible phase, wherein said hydrogel microspheres are isolated from each other by said water-immiscible liquid phase such that a flow and/or exchange of aqueous sample between different hydrogel microspheres is not possible and does not occur anymore;

d) performing an enzymatic amplification protocol on said hydrogel microspheres, wherein said enzymatic amplification protocol generates a first optically detectable signal in a hydrogel microsphere being associated with or comprising said analyte; and wherein said enzymatic amplification protocol generates a second optically detectable signal in a hydrogel microsphere not being associated with or not comprising said analyte;

e) determining, by detecting said first and second optically detectable signals, respectively, a number $N_{pos}$ of

hydrogel microspheres having a first optically detectable signal (= "positive" hydrogel microspheres) and a number $N_{neg}$ of hydrogel microspheres having a second optically detectable signal (= "negative" hydrogel microspheres); wherein the total number of detected hydrogel microspheres $N = N_{pos} + N_{neg}$;

f) calculating a concentration $c_s$ of said analyte in said sample in accordance with formula

$$c_S = \frac{N_B}{V_S} \cdot -ln\left(\frac{N_{neg}}{N}\right) = \frac{N_B}{V_S} \cdot \lambda;$$

wherein

$c_S$ = concentration of analyte in aqueous sample
$N_B$ = pre-defined number of hydrogel microspheres exposed to aqueous sample
$V_S$ = volume of aqueous sample containing, or suspected of containing, analyte
$N_{neg}$ = number of detected hydrogel microspheres having a second optically detectable signal (= "negative" hydrogel microspheres)
$N$ = number of all detected hydrogel microspheres = $N_{neg} + N_{pos}$.
wherein
$N_{pos}$ = number of detected hydrogel microspheres having a first optically detectable signal (="positive" hydrogel microspheres).

3.  The method according to any of claims 1 - 2, wherein said analyte is:

a) a target nucleic acid; and said enzymatic amplification protocol is a target amplification reaction; or
b) a target protein; and said enzymatic amplification protocol is a signal amplification reaction; or
c) a biological cell or a viral particle or an extracellular vesicle, each comprising various types of nucleic acid within said cell, viral particle or extracellular vesicle; and said enzymatic amplification protocol is a target amplification reaction.

4.  The method according to any of claims 1, 2 - 3, wherein said analyte is:

a) a target nucleic acid; and said enzymatic amplification protocol is a nucleic acid amplification protocol resulting in specific amplification of said target nucleic acid, if associated with or comprised by any of said hydrogel microspheres, and resulting in a generation of a first optically detectable signal in a hydrogel microsphere, if said hydrogel microsphere comprises or is associated with amplified target nucleic acid; or
b) a target protein; and said enzymatic amplification protocol is a signal amplification reaction involving the formation of an analyte-specific immune-complex on or in a hydrogel microsphere, if said hydrogel microsphere is associated with or comprises said analyte; wherein a chromogenic enzyme is attached to said analyte-specific immune complex, and wherein said enzymatic amplification reaction, through the action of said chromogenic enzyme, furthermore involves an accumulation of an optically detectable product, resulting in a generation of a first optically detectable signal in a hydrogel microsphere, if said hydrogel microsphere comprises or is associated with said analyte; or
c) a target protein; and said enzymatic amplification protocol is a signal amplification reaction involving the formation of an analyte-specific immune-complex on or in a hydrogel microsphere, if said hydrogel microsphere is associated with or comprises said analyte; wherein a nucleic acid label is attached to said analyte-specific immune complex, and wherein said enzymatic amplification reaction furthermore involves a specific nucleic acid amplification of said nucleic acid label, resulting in an accumulation of amplified nucleic acid label and a generation of a first optically detectable signal in a hydrogel microsphere, if said hydrogel microsphere comprises or is associated with said analyte; or
d) a biological cell or a viral particle or an extracellular vesicle, each comprising various types of nucleic acid within said cell, viral particle or extracellular vesicle; and said enzymatic amplification protocol is a nucleic acid amplification protocol resulting in specific amplification of one or several types of nucleic acid comprised within said biological cell, viral particle or extracellular vesicle, if said biological cell, viral particle or extracellular vesicle is associated with or comprised by any of said hydrogel microspheres, and resulting in a generation of a first optically detectable signal in a hydrogel microsphere, if said hydrogel microsphere comprises or is associated with a biological cell, viral particle or extracellular vesicle.

5.  The method according to any of claims 1, 2 - 4, wherein said material capable of binding said analyte is selected from:

a) cationic polymers, cationic oligomers, cationic monomers, and silica, if the analyte is a nucleic acid, and if said material capable of binding said analyte non-specifically binds nucleic acids;

b) oligonucleotides, if the analyte is a nucleic acid, and if said material capable of binding said analyte specifically binds a particular nucleic acid, e.g. a target nucleic acid; wherein for a given nucleic acid analyte, said oligonucleotide is complementary thereto;

c) antibodies, antibody fragments, and protein receptors, if the analyte is a protein, a biological cell, a viral particle or an extracellular vesicle, and if said material capable of binding said analyte specifically binds a particular protein, e.g. a target protein, or specifically binds a label, tag, prosthetic group or other component associated with said analyte or associated with an antibody, antibody fragment or protein receptor that specifically binds to said analyte.

6. The method according to any of claims 1, 2 - 5, in particular claim 5, wherein said analyte is a nucleic acid, said material capable of binding said analyte binds nucleic acids non-specifically; and wherein said material capable of non-specifically binding nucleic acids is selected from:
chitosan and derivatives thereof, gelatin and derivatives thereof, poly(ethyleneimine), poly(2-dimethyl(aminoethyl)methacrylate), poly(lysine), poly(histidine), poly(arginine) and polymer backbones having basic amino acids attached or incorporated as part of such backbone; oligopeptides comprising or consisting of basic amino acid, such as histidine, lysine, and arginine; and monomers selected from basic amino acids, such as histidine, lysine, and arginine.

7. The method according to any of claims 1, 2 - 6, wherein said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000.

8. The method according to any of claims 1, 2 - 7, wherein said hydrogel microspheres comprise magnetic particles allowing for mechanical handling and transfer of said hydrogel microspheres.

9. The method according to any of claims 1, 2 - 8, wherein said method comprises an additional step b*) which is performed after step b) and before step c):
b*) washing said pre-defined number $N_B$ of hydrogel microspheres, by exposing them to a wash buffer.

10. The method according to any of claims 1, 2 - 9, wherein said method comprises an additional step b**) which is performed

- after step b) and before step c), and,
- if additionally step b*) is performed according to claim 9, then step b**) is performed after step b*) and before step c):
b**) processing said pre-defined number $N_B$ of hydrogel microspheres by exposing them to a solution for performing said enzymatic amplification reaction, said solution comprising

(i) a buffer, mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; or

(ii) a buffer, an analyte-specific antibody or antibody-fragment having a label attached, and a detection reagent for detection of said label; or

(iii) a buffer, an analyte-specific antibody or antibody fragment, a detection antibody for detecting analyte bound to said analyte-specific antibody or antibody fragment, said detection antibody having a label attached, and a detection reagent for detection of said label;

wherein in (ii) and (iii), said label is either a nucleic acid tag or a chromogenic enzyme; and,

- if said label is a nucleic acid tag, said detection reagent is a solution comprising mono-nucleoside triphosphates, an amplification enzyme, a nucleic acid dye for the detection of an amplified nucleic acid, optionally one or more amplification primers or one or more pairs of amplification primers, if not already comprised by said hydrogel microspheres, and further optionally a molecular probe, such as a TaqMan Probe, or a molecular beacon; and,

- if said label is a chromogenic enzyme, said detection reagent is a solution comprising a substrate for said chromogenic enzyme.

11. The method according to any of claims 1, 2 - 10, wherein, in step e), said first optically detectable signal generated in said hydrogel microspheres, and optionally also said second optically detectable signal, is detected via a first channel, preferably fluorescence channel, and wherein, further optionally, the total number of detected microspheres $N$ is additionally determined by optical imaging, via a second channel.

12. The method according to any of claims 1, 2 - 11, wherein said hydrogel microspheres have an average diameter in a range of from 10 $\mu$m to 500 $\mu$m, preferably from 20 $\mu$m to 500 $\mu$m, more preferably from 20 $\mu$m to 200 $\mu$m, more preferably from 20 $\mu$m to 100 $\mu$m, and even more preferably from 40 $\mu$m to 100 $\mu$m.

13. A method of quantifying a concentration $c_s$ of a target nucleic acid in a sample, in particular according to any of the foregoing claims, said method comprising the steps:

a) Providing, in any order, an aqueous sample containing, or suspected of containing, a target nucleic acid, and an aqueous suspension of a pre-defined number $N_B$ of hydrogel microspheres, said hydrogel microspheres comprising a material capable of non-specifically binding nucleic acids, including said target nucleic acid, or specifically binding said target nucleic acid;

b) allowing said hydrogel microspheres to take up aqueous sample and to bind said target nucleic acid, preferably all of said target nucleic acid, if present in said aqueous sample, by exposing said pre-defined number $N_B$ of hydrogel microspheres to said aqueous sample and mixing said aqueous sample with said suspension of hydrogel microspheres; thus resulting in at least some hydrogel microspheres of said pre-defined number $N_B$ of hydrogel microspheres being associated with or comprising said target nucleic acid contained by said aqueous sample provided in step a);

c) transferring said hydrogel microspheres into a liquid phase that is immiscible with water and aqueous solutions, e. g. an oil-phase, thereby generating a suspension of said pre-defined number $N_B$ of hydrogel microspheres in said water-immiscible phase, wherein said hydrogel microspheres are isolated from each other by said water-immiscible liquid phase such that a flow and/or exchange of aqueous sample between different hydrogel microspheres is not possible and does not occur anymore;

d) performing a nucleic acid amplification protocol on said hydrogel microspheres that is specific for said target nucleic acid; wherein said nucleic acid amplification protocol results in specific amplification of said target nucleic acid if associated with or comprised by any of said hydrogel microspheres, and resulting in a generation of a first optically detectable signal in a hydrogel microsphere, if said hydrogel microsphere comprises or is associated with amplified target nucleic acid; and wherein said nucleic acid amplification protocol results in no amplification of said target nucleic acid and in a generation of a second optically detectable signal in a hydrogel microsphere, if said hydrogel microsphere is not associated with and does not comprise amplified target nucleic acid;

e) determining, by detecting said first and second optically detectable signals, respectively, a number $N_{pos}$ of hydrogel microspheres having a first optically detectable signal (= "positive" hydrogel microspheres) and a number $N_{neg}$ of hydrogel microspheres having a second optically detectable signal (= "negative" hydrogel microspheres); wherein the total number of detected microspheres $N = N_{pos} + N_{neg}$;

f) calculating a concentration $c_s$ of a target nucleic acid in said aqueous sample in accordance with formula

$$c_S = \frac{N_B}{V_S} \cdot -ln\left(\frac{N_{neg}}{N}\right) = \frac{N_B}{V_S} \cdot \lambda;$$

wherein

$c_s$ = concentration of target nucleic acid in aqueous sample
$N_B$ = pre-defined number of hydrogel microspheres exposed to aqueous sample
$V_S$ = volume of aqueous sample containing, or suspected of containing, target nucleic acid
$N_{neg}$ = number of detected hydrogel microspheres having a second optically detectable signal (= "negative" hydrogel microspheres)
$N$ = number of all detected hydrogel microspheres = $N_{neg} + N_{pos}$;
wherein
$N_{pos}$ = number of detected hydrogel microspheres having a first optically detectable signal (="positive" hydrogel microspheres).

**14.** An aqueous suspension of a pre-defined number $N_B$ of hydrogel microspheres for use in a method of quantifying a concentration $c_s$ of an analyte, preferably of a target nucleic acid, in a sample according to any of claims 2 - 13, said aqueous suspension comprising an aqueous solvent and a pre-defined number $N_B$ hydrogel microspheres, wherein said hydrogel microspheres comprise a material capable of binding an analyte, preferably capable of binding a nucleic acid.

**15.** The aqueous suspension according to claim 14, wherein said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000.

**16.** A macroparticle for use in a method of quantifying a concentration $c_s$ of an analyte, preferably of a target nucleic acid, in a sample according to any of claims 1 - 13, said macroparticle comprising a matrix and a pre-defined number $N_B$ of hydrogel microspheres embedded in said matrix of said macroparticle, wherein said macroparticle is dried, preferably freeze-dried.

**17.** The macroparticle according to claim 16, wherein said pre-defined number $N_B$ of hydrogel microspheres is in a range of from 1,000 to 1,000,000, preferably in a range of from 5,000 to 500,000, more preferably in a range of from 5,000 to 100,000, even more preferably in a range of from 5,000 to 50,000, and even more preferably in a range of from 5,000 to 20,000

**18.** The macroparticle according to any of claims 16 cand 17, wherein said matrix comprises or is made of a material that is an excipient for drying processes, in particular for freeze drying.

**19.** The macroparticle according to claim 18, wherein said excipient for drying, in particular for freeze drying, is selected from saccharides, such as trehalose, sucrose, mannitol, glucose, fructose, lactose, mannitol, inositol, hydroxypropyl-β-cyclodextrin and combinations thereof; polymers such as polyethyleneglycol (PEG), polyvinylpyrrolidone (PVP), dextran, gelatin; amino acids, such as arginine, histidine, and glycine; and combinations of any of the foregoing

**20.** The macroparticle according to any of claims 16 - 19, which is substantially spherical, globular, droplet-shaped, ellipsoidal or otherwise round-shaped and has an average diameter in a range of from 1 mm to 50 mm, preferably from 2 mm to 20 mm, more preferably from 2 mm to 15 mm, and even more preferably from 2 mm to 10 mm, and even more preferably from 2 mm to 5 mm.

**21.** A method of generating a macroparticle according to any of claims 16 - 20, said method comprising the steps:

a) providing, in any order, a suspension of a predefined number $N_B$ of hydrogel microspheres, as defined in any of claims 2 - 13, an aqueous solvent and at least one matrix material as defined in any of claims 18 and 19;
b) adding said at least one matrix material to said aqueous solvent, thereby dissolving said matrix material in said aqueous solvent, and mixing said solution of matrix material with said suspension of a predefined number $N_B$ of hydrogel microspheres, thereby forming a suspension of said hydrogel microspheres in said aqueous solvent;
c) generating droplets of said suspension formed in step b), such droplets having a defined size and volume, and dispensing such droplets into a water-immiscible liquid phase, e.g. an oil phase, that is at a temperature in a range of from -100°C to -10°C, preferably from -90°C to -20°C, more preferably from -80°C to -30°C; thereby allowing said droplets to freeze;
d) separating said frozen droplets from said water-immiscible liquid phase and drying, preferably freeze-drying, them, thus resulting in substantially spherical, globular, droplet-shaped, ellipsoidal or otherwise round-shaped macroparticles according to any of claims 16 - 20.

**22.** A method of generating an aqueous suspension of a pre-defined number $N_B$ of hydrogel microspheres according to any of claims 14 - 15, said method comprising the step:

a) Providing a macroparticle according to any of claims 16 - 20 and dissolving such macroparticle in an aqueous solvent; or
a*) providing a predefined number $N_B$ of hydrogel microspheres, as defined in any of claims 2 - 13, in dried form, and suspending them in an aqueous solvent.

VS = volume of sample containing analyte

Individual pre-made reaction compartment with material capable of binding analyte

detected reaction compartments having a first optically detectable signal; e.g. appearing bright and therefore having an optically detectable signal

NB = pre-defined number of reaction compartments exposed to the sample

detected reaction compartment having a second optically detectable signal, e.g. appearing dark and therefore without an optically detectable signal

**Figure 1**

**Figure 2**

**Figure 3**

a.

b.

| | |
|---|---|

Determine Sample Volume & microsphere Count

- (optional) wash steps

Incubation with PCR Reagents

Suspension in Oil

Target Amplification & Fluorescence Detection

Apply Formula & report Analyte Concentration in Sample

**Figure 4**

Determine Sample Volume & microsphere Count

Enzyme

- (optional) wash steps

Incubation with Chromogenic Enzyme Substrate

Suspension in Oil

Enzymatic Substrate Conversion & Fluorescence Detection

Apply Formula & report Analyte Concentration in Sample

**Figure 5**

Figure 6

EP 4 438 737 A1

**Figure 7**

EP 4 438 737 A1

Hydrogel Microsphere Suspension (known concentration )

Solution of matrix material in aqueous solvent

Generate Droplets

Introduce Droplets in cold oil, e.g. FC oil

Freeze dry frozen Droplets

Collect Pellets (= "macroparticles")

**Figure 8**

Macroparticle Matrix

Embedded hydrogel microsphere

edge of water droplet with dissolved macro-particle and resuspending hydrogel microspheres

3mm

3mm

Macroparticle, broken in half. Imaged under dark field illumination conditions. Embedded hydrogel microspheres visible as dark spots.

After addition of water to macroparticle, the matrix is dissolved and hydrogel microspheres are re-suspended. hydrogel microspheres are visible as bright spots.

**Figure 9**

EP 4 438 737 A1

```
┌─────────────────────┐
│                     │
│   Macroparticle     │─────────────┐
│                     │             │
│                     │             ▼
└─────────────────────┘   ┌─────────────────────┐          ┌─────────────────────┐
                          │  Suspension with    │          │                     │
                          │  known Number of    │- - - - ->│ hydrogel microsphere│
                          │ hydrogel microspheres│         │       Assay         │
                          │                     │          │                     │
┌─────────────────────┐   └─────────────────────┘          └─────────────────────┘
│                     │             ▲
│      Buffer         │─────────────┘
│                     │
│                     │
└─────────────────────┘
```

**Figure 10**

**Calculating Target Concentration
in conventional dPCR**

$$c_S = \frac{D}{V_C} \cdot -\ln\left(\frac{N_{neg}}{N}\right) = \frac{D}{V_C} \cdot \lambda$$

$C_S$ – Target Concentration in Sample
$N_{neg}$ – number of PCR negative compartments
N - total number of compartments
$V_C$ - volume of a single droplet or compartment,
D - dilution factor

**Calculating Concentration
with hydrogel microspheres as an example
of suitable reaction compartments**

$$c_S = \frac{N_B}{V_S} \cdot -ln\left(\frac{N_{neg}}{N}\right) = \frac{N_B}{V_S} \cdot \lambda$$

$C_s$ – Target Concentration in Sample
$N_{neg}$ – number of negative hydrogel microspheres
N - total number of hydrogel microspheres
$N_B$ - the total number of hydrogel microspheres
applied to the sample
$V_S$ – sample volume

**Figure 11**

**Figure 12**

| Dilution Level | Prop. Neg. Beads Mean [-] | No. Beads $N$ Mean | Lambda $\lambda$ Mean [cp/Bead] | Target Conc. $c_S$ Mean [cp/µL] | Target Conc. $c_S$ Log10 Mean [log10 cp/µL] | Target Conc. cS Log10 Std [log10 cp/µL] |
|---|---|---|---|---|---|---|
| V1 | 0.01081 | 3145 | 4.5386 | 25643.08 | 4.41 | 0.02 |
| V2 | 0.51874 | 3371 | 0.66024 | 3730.37 | 3.57 | 0.01 |
| V3 | 0.93291 | 2933 | 0.06960 | 393.21 | 2.59 | 0.02 |
| V4 | 0.99418 | 3500 | 0.00582 | 32.89 | 1.51 | 0.11 |
| V5* | 0.99988 | 3385 | 0.00012 | 2.10** | 0.30** | 0.16** |

**Figure 13**

**Figure 14**

Europäisches
Patentamt

European
Patent Office

Office européen
des brevets

**EUROPEAN SEARCH REPORT**

Application Number

EP 23 16 6156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | HEINRICH THERESA ET AL: "DNA-Binding Magnetic Nanoreactor Beads for Digital PCR Analysis", ANALYTICAL CHEMISTRY, 30 August 2023 (2023-08-30), XP093083096, US ISSN: 0003-2700, DOI: 10.1021/acs.analchem.3c01418 Retrieved from the Internet: URL:https://pubs.acs.org/doi/pdf/10.1021/acs.analchem.3c01418> ----- | | INV. C12Q1/6804 C12Q1/6834 |
| X | LONCAREVIC IVAN FRANCISCO ET AL: "Ultra-precise quantification of mRNA targets across a broad dynamic range with nanoreactor beads", PLOS ONE, vol. 16, no. 3, 18 March 2021 (2021-03-18), page e0242529, XP093083149, DOI: 10.1371/journal.pone.0242529 | 14 | |
| Y | * the whole document * ----- | 1-13, 15-22 | TECHNICAL FIELDS SEARCHED (IPC) C12Q |
| X | ZHU YANZHE ET AL: "Single-Cell Phenotypic Analysis and Digital Molecular Detection Linkable by a Hydrogel Bead-Based Platform", ACS APPLIED BIO MATERIALS, vol. 4, no. 3, 12 February 2021 (2021-02-12), pages 2664-2674, XP093082927, US ISSN: 2576-6422, DOI: 10.1021/acsabm.0c01615 Retrieved from the Internet: URL:https://pubs.acs.org/doi/pdf/10.1021/acsabm.0c01615> * the whole document * ----- -/-- | 1-22 | |

The present search report has been drawn up for all claims

| Place of search | Date of completion of the search | Examiner |
|---|---|---|
| The Hague | 20 September 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

**EUROPEAN SEARCH REPORT**

Europäisches
Patentamt
European
Patent Office
Office européen
des brevets

Application Number

EP 23 16 6156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 839 508 A1 (BLINK AG [DE]) 23 June 2021 (2021-06-23) | 14-22 | |
| Y | * claims 1-27 * <br> * paragraphs [0079] - [0080], [0082] * <br> * examples 1-3 * | 1-13 | |
| Y | WO 2019/089996 A1 (UNIV WASHINGTON [US]) 9 May 2019 (2019-05-09) <br> * claims 1-96 * <br> * paragraphs [0090] - [0091], [0314] - [0344], [0355] - [0367], [0370] - [0383], [0403] * | 1-22 | |
| Y | Bio-Rad: "Droplet Digital PCR Applications Guide", <br> , <br> 1 February 2010 (2010-02-01), XP055645030, Retrieved from the Internet: URL:http://www.bio-rad.com/webroot/web/pdf /lsr/literature/Bulletin_6407.pdf [retrieved on 2019-11-21] <br> * the whole document * <br> * page 84, paragraph 5 - page 85; figure 6.1 * <br> * page 85; figure 7.7 * <br> * pages 8,49-50, * <br> * pages 52-56 * | 1-22 | TECHNICAL FIELDS SEARCHED (IPC) |
| Y | WO 2012/100198 A2 (UNIV WASHINGTON CT COMMERCIALI [US]; CHIU DANIEL T [US] ET AL.) 26 July 2012 (2012-07-26) <br> * paragraphs [0048], [0068] - [0069] * <br> * claims 1-30 * | 1-22 | |

-/--

The present search report has been drawn up for all claims

| Place of search | Date of completion of the search | Examiner |
|---|---|---|
| The Hague | 20 September 2023 | Bruma, Anja |

EPO FORM 1503 03.82 (P04C01)

page 2 of 3

# EP 4 438 737 A1

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/135667 A1 (LIFE TECHNOLOGIES CORP [US]; JANAWAY GORDON [US] ET AL.) 4 October 2012 (2012-10-04) * claims 1-11 * * paragraphs [0002] – [0008], [0033] – [0042], [0095], [0108] – [0122], [0122] – [0142] * ----- | 1-22 | |
| | | | TECHNICAL FIELDS SEARCHED (IPC) |

The present search report has been drawn up for all claims

| Place of search | Date of completion of the search | Examiner |
|---|---|---|
| The Hague | 20 September 2023 | Bruma, Anja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 16 6156

20-09-2023

| Patent document cited in search report | | | Publication date | Patent family member(s) | | | Publication date |
|---|---|---|---|---|---|---|---|
| EP 3839508 | A1 | | 23-06-2021 | AU | 2020403853 | A1 | 07-07-2022 |
| | | | | BR | 112022011676 | A2 | 11-10-2022 |
| | | | | CA | 3161939 | A1 | 14-06-2022 |
| | | | | CN | 115151819 | A | 04-10-2022 |
| | | | | EP | 3839508 | A1 | 23-06-2021 |
| | | | | EP | 4078181 | A1 | 26-10-2022 |
| | | | | IL | 293851 | A | 01-08-2022 |
| | | | | JP | 2023506261 | A | 15-02-2023 |
| | | | | KR | 20220114630 | A | 17-08-2022 |
| | | | | US | 2023063987 | A1 | 02-03-2023 |
| | | | | WO | 2021122579 | A1 | 24-06-2021 |
| WO 2019089996 | A1 | | 09-05-2019 | CN | 111683897 | A | 18-09-2020 |
| | | | | EP | 3704059 | A1 | 09-09-2020 |
| | | | | JP | 7333634 | B2 | 25-08-2023 |
| | | | | JP | 2021501597 | A | 21-01-2021 |
| | | | | US | 2020362391 | A1 | 19-11-2020 |
| | | | | WO | 2019089996 | A1 | 09-05-2019 |
| WO 2012100198 | A2 | | 26-07-2012 | CN | 103429997 | A | 04-12-2013 |
| | | | | CN | 107502660 | A | 22-12-2017 |
| | | | | EP | 2665995 | A2 | 27-11-2013 |
| | | | | EP | 3410080 | A1 | 05-12-2018 |
| | | | | JP | 6063395 | B2 | 18-01-2017 |
| | | | | JP | 6426679 | B2 | 21-11-2018 |
| | | | | JP | 6654680 | B2 | 26-02-2020 |
| | | | | JP | 2014505476 | A | 06-03-2014 |
| | | | | JP | 2017062250 | A | 30-03-2017 |
| | | | | JP | 2019030323 | A | 28-02-2019 |
| | | | | US | 2014087386 | A1 | 27-03-2014 |
| | | | | US | 2017145490 | A1 | 25-05-2017 |
| | | | | US | 2018251827 | A1 | 06-09-2018 |
| | | | | US | 2020157615 | A1 | 21-05-2020 |
| | | | | WO | 2012100198 | A2 | 26-07-2012 |
| WO 2012135667 | A1 | | 04-10-2012 | EP | 2694673 | A1 | 12-02-2014 |
| | | | | US | 2014248623 | A1 | 04-09-2014 |
| | | | | US | 2016208342 | A1 | 21-07-2016 |
| | | | | US | 2020224280 | A1 | 16-07-2020 |
| | | | | US | 2023122742 | A1 | 20-04-2023 |
| | | | | WO | 2012135667 | A1 | 04-10-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2022080978 W

- EP 22165559

**Non-patent literature cited in the description**

- **HINDSON et al.** *Anal. Chem.,* 2011, vol. 83 (22), 8604-8610
- **GENG et al.** *Anal. Chem.,* 2014, vol. 86, 703-712
- **LENG et al.** *Lab Chip.,* 2010, vol. 10, 2481-2843
- **LONCAREVIC et al.** *Plos One,* 2021, vol. 16, e0242529
- **M. SPENGLER ; M. ADLER ; C.M. NIEMEYER.** Highly sensitive ligand-binding assays in pre-clinical and clinical applications: immuno-PCR and other emerging techniques. *Analyst.,* 2015, vol. 140, 6175-6194, https://doi.ors/10.1039/c5an00822k
- **L. CHANG ; J. LI ; L. WANG.** Immuno-PCR: An ultrasensitive immunoassay for biomolecular detection. *Anal Chim Acta.,* 2016, vol. 910, 12-24, https://doi.org/10.1016/j.aca.2015.12.039

- **H. SCHRÖDER ; M. GRÖSCHE ; M. ADLER ; M. SPENGLER ; C.M. NIEMEYER.** Immuno-PCR with digital readout. *Biochem Bioph Res Co.,* 2017, vol. 488, 311-315, https://doi.org/10.1016/j.bbrc.2017.04.162
- **D.M. RISSIN ; C.W. KAN ; T.G. CAMPBELL ; S.C. HOWES ; D.R. FOURNIER ; L. SONG ; T. PIECH ; P.P. PATEL ; L. CHANG ; A.J. RIVNAK.** Single-Molecule enzyme-linked immunosorbent assay detects serum proteins at subfemtomolar concentrations. *Nat Biotechnol.,* 2010, vol. 28, 595-599, https://doi.org/10.1038/nbt.1641
- **S.M. SCHUBERT ; L.M. ARENDT ; W. ZHOU ; S. BAIG ; S.R. WALTER ; R.J. BUCHSBAUM ; C. KUPERWASSER ; D.R. WALT.** Ultra-sensitive protein detection via Single Molecule Arrays towards early stage cancer monitoring. *Sci Rep-Uk,* 2015, vol. 5, 11034, https://doi.org/10.1038/srep11034